# EUROPEAN PATENT APPLICATION

(11) **EP 1 897 885 A1**
(43) Date of publication of application: **12.03.2008**
(21) Application number: 06090166.7
(22) Date of filing: 08.09.2006
(51) Int. Cl.: C07K 1/00

(54) **Compounds and methods for F labelled agents**

(71) Applicant: Bayer Schering Pharma Aktiengesellschaft, 13353 Berlin (DE)
(72) Inventor: Lehmann, Lutz, 13357 Berlin (DE); Srinivasan, Ananth, 10629 Berlin (DE); Brumby, Thomas, 12163 Berlin (DE); Sülzle, Detlef, 13467 Berlin (DE); Stellfeld, Timo, 14197 Berlin (DE); Graham, Keith, 10115 Berlin (DE); Karramkam, Mylene, 97122 Baie-Mahault (GP); Ametamey, Simon, 8045 Zürich (CH)

(57) **Abstract**

This invention relates to novel compounds suitable for or already radiolabeled with ¹⁸F, methods of making such compounds and use of such compounds for diagnostic imaging. Such labeled compounds are characterized by Formula 11, wherein the substituents G, L, U, and Q have the meaning as defined in the specification.

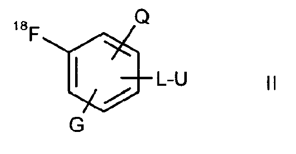

## Description

### Background of the Invention:

### Field of invention

This invention relates to novel compounds suitable for or already radiolabeled with ¹⁸F, methods of making such compounds and use of such compounds for diagnostic imaging.

### Background art

The nucleophilic aromatic ¹⁸F-fluorination reaction is of great importance for ¹⁸F-labeled radiopharmaceuticals which are used as *in vivo* imaging agents targeting and visualizing diseases, e.g. solid tumors or diseases of brain. A very important technical goal in using ¹⁸F-labeled radiopharmaceuticals is the quick preparation and administration of the radioactive compound due to the fact that the ¹⁸F isotopes have a short half-life of about only 111 minutes.

The conversions of *mono*- (mainly para-) substituted trimethylammonium benzene derivatives (**1**) to substituted [¹⁸F]-fluoro-benzene derivatives (**2**) which may serve as radiopharmaceutical itself or as prosthetic group for the F-18 labeling of small and large molecules have been reported in the literature (Irie et al. 1982, Fluorine Chem., 27, (1985), 117-191; Haka et al. 1989) (see scheme 1).

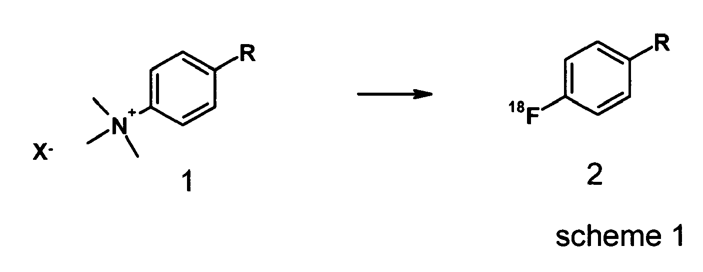

There are only a few publications about nucleophilic aromatic ¹⁸F-fluorination reactions of trimethyl ammonium substituted aromatic derivatives which contain two or more substituents beside the trimethylammonium moiety:
Oya et al. treated [2-Chloro-5-(2-dimethylcarbamoyl-phenylsulfanyl)-4-nitrophenyl]-trimethyl-ammonium triflate with [18F] potassium fluoride and obtained the desired 18F-labeled compound (Journal of Medicinal Chemistry (2002), 45(21), 4716-4723).
Li et al. reported on the 18F-fluorination reaction of 4-(N,N,N-trimethylammonium)-3-cyano-3'-iodobenzophenone triflate (Bioconjugate Chemistry (2003), 14(2), 287-294).
Enas et al. converted (2,2-Dimethyl-1,3-dioxo-indan-5-yl)-trimethyl-ammonium triflate into the desired 18F-labeled compound (Journal of Fluorine Chemistry (1993), 63(3), 233-41).
Seimbille et al. and other groups labeled (2-Chloro-4-nitro-phenyl)-trimethyl-ammonium triflate successfully with 18F (J. Labeled Compd. Radiopharm., (2005), 48, 11, 829-843).

(2-Benzyloxy-4-formyl-phenyl)-trimethyl-ammonium triflate was successfully labeled with ¹⁸F at high temperature (130° C) by Langer et. al. (Bioorg. Med. Chem.; EN; 9; 3; 2001; 677 - 694).

Lang et al. radiolabeled trimethyl-(2-methyl-4-pentamethylphenyl methoxycarbonyl-phenyl)-ammonium triflate by use of [18F] potassium fluoride (J. Med. Chem., 42; 9; 1999; 1576 - 1586).

Trimethyl-(4-nitro-naphthalen-1-yl)-ammonium triflate was labeled with ¹⁸F by Amokhtari et al. (J. Labeled Compd. Radiopharm.; S42; 1; (1999); S622 - S623).

Lemaire et al. converted (2-formyl-5-methoxy-phenyl)-trimethyl-ammonium triflate into the desired 18F-labeled product (J. Labeled Compd. Radiopharm.; 44; 2001; S857 - S859).

VanBrocklin et al. described the 18F labeling of (2-bromo-4-nitro-phenyl)-trimethyl-ammonium triflate (J. Labeled Compd. Radiopharm., 44; 2001; S880 - S882) and

Cetir Centre Medic reported on the successful ¹⁸F-labeling of (5-Chloro-8-hydroxy-quinolin-7-yl)-trimethyl-ammonium triflate (EP1563852 A1).
Most of these mentioned ¹⁸F-labeled aromatic derivatives which contain two or more additional substituents can not be coupled to chemical functionalities like amines, thiols, carboxylic acids, phenols or other chemicals groups of complex molecules like peptides without further transformations.
¹⁸F labeling of more complex radiopharmaceuticals like peptides takes place in all known publications in a two- or multi-step strategy (see scheme 2, review article: Eur. J. Nucl. Med. (2001), 28, 929-938).
For these kinds of ¹⁸F-labeling also mono-substituted trimethylammonium benzene derivatives are used and react in a first step with [¹⁸F] potassium fluoride to obtain substituted [¹⁸F]-fluoro-benzene derivatives. These compounds are then coupled in a second step to larger and more complex molecules like peptides, small molecules or nucleotides (see scheme 2). 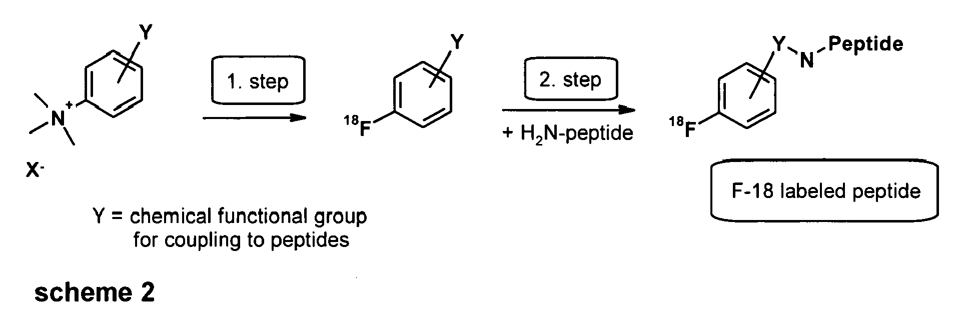 Especially 4-[¹⁸F]fluorobenzaldehyde has been used in many examples for F-18 labelling of complex molecules (e.g. Journal of Nuclear Medicine (2004), 45(5), 892-902). But also N-succinimidyl-8-[4'-[¹⁸F]fluorobenzylamino]suberate (Bioconjugate Chem., (1991), 2, 44-49), 4-[¹⁸F]fluorophenacyl bromide and 3-[¹⁸F]fluoro-5-nitrobenzimidate (J. Nucl. Med., (1987), 28, 462-470), m-maleimido-N-(p-[¹⁸F]fluorobenzyl)-benzamide (J. Labeled Compd. Radiopharm (1989), 26, 287-289,), *N-*{4-[4-[¹⁸F]fluorobenzylidene(aminooxy)-butyl}-maleimide (Bioconjugate Chem., (2003), 14, 1253-1259), [¹⁸F]*N*-(4-fluorobenzyl)-2-bromoacetamide (Bioconjugate Chem., (2000), 11, 627-636) and [¹⁸F]-3,5-difluorophenyl azide (and 5 derivatives) (J. Org. Chem., (1995), 60, 6680-6681) are known examples. F-18 labelling of peptides via para-[18F]-fluorobenzoates is also a very common method either by coupling of the corresponding acid with additional activating agents (such as 1,3-dicyclohexylcarbodiimide/1-hydroxy-7-azabenzotriazole (DCC/HOAt) or N-[(dimethylamino)-1H-1,2,3-triazolyl[4,5]pyridine-1-yl-methylene]-N-methyl-methanaminium hexafluorophosphate N-oxide (HATU/DIPEA, Eur. J. Nucl. Med. Mol. Imaging., (2002), 29, 754-759) or by isolated N-succinimidyl 4-[¹⁸F]fluorobenzoate (Nucl. Med. Biol. (1996), 23, 365).

None of these compounds and none of other published compounds allow a direct (one-step) labelling of peptides with ¹⁸F-fluoride.

Object of the present invention is the development of a practical and mild technique for ¹⁸F labeling of molecules like e.g. peptides, oligonucleotides or small molecules.

### Summary of the Invention:

The object of the present invention is solved as detailed below.

The present invention provides novel compounds of Formulae I and II.

The present invention also provides diagnostic compositions comprising a radiolabeled compound of Formula I or II and a pharmaceutically acceptable carrier or diluent.

The invention further provides a method of imaging diseases, the method comprising introducing into a patient a detectable quantity of a labeled compound of Formula II or pharmaceutically acceptable salt, ester, amide or prodrug thereof.

The invention provides the compounds of Formula II for use as medicament.

Another aspect of the invention is directed to the use of compounds of Formula I or II for the manufacture of a medicament.

The present invention also provides a kit for preparing a radiopharmaceutical preparation, said kit comprising a sealed vial containing a predetermined quantity of the compound of Formula I.

A further aspect of this invention is directed to methods and intermediates useful for synthesizing the tumor imaging compounds of Formula I and II described herein.

More specifically the compounds of this invention are useful for the imaging of a variety of cancers including but not limited to: carcinoma such as bladder, breast, colon, kidney, liver, lung, including small cell lung cancer, esophagus, gall-bladder, ovary, pancreas, stomach, cervix, thyroid, prostate, and skin, hematopoetic tumors of lymphoid and myeloid lineage, tumors of mesenchymal origin, tumors of central peripheral nervous systems, other tumors, including melanoma, seminoma, teratocarcinoma, osteosarcoma, xeroderma pigmentosum, keratoxanthoma, thyroid follicular cancer, and Karposi's sarcoma.

### Detailed Description of the Invention:

In a first aspect the present invention is directed to compounds of Formula **I** 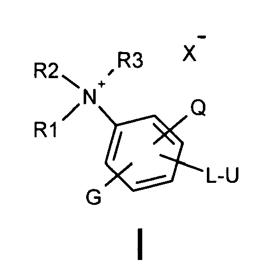 wherein:
**L** is a bond, -CO-, -SO₂-, -(CH₂)_{d}-CO-, -SO-, or -C≡CCO-, wherein the respective substituent can be in ortho, meta or para-position and d is an integer between 1 and 6.

In preferred embodiments of compounds of Formula I, **L** is -CO-, -SO₂-, or -C≡C-CO-, wherein the respective substituent can be in meta or para position.

In further preferred embodiments of compounds of Formula I, **L** is -CO-, or -SO₂, wherein the respective substituent can be in meta or para position.

**G** is selected from -F, -Cl, -Br, -I, -NO, -NO₂, -NR⁴COCF₃, -NR⁴SO₂CF₃, -N(CF₃)₂, -NHCSNHR⁴, -N(SO₂R⁵)₂, -N(O)=NCONH₂, -NR⁴CN, -NHCSR⁵, -N≡C, -N=C(CF₃)₂, -N=NCF₃, -N=NCN, -NR⁴COR⁴, -NR⁴COOR⁵, -OSO₂CF₃, -OSO₂C₆H₅, -OCOR⁵, -ONO₂, -OSO₂R⁵, -O-C=CH₂, -OCF₂CF₃, -OCOCF₃, -OCN, -OCF₃, -C=N, -C(NO₂)₃, -COOR⁴, -CONR⁴R⁵, -CSNH₂, -CH=NOR⁴, -CH₂SO₂R⁴, -COCF₃, -CF₃, -CF₂Cl-CBr₃, -CCIF₂, -CCl₃, -CF₂CF₃, -C≡CR⁴, -CH=NSO₂CF₃, -CH₂CF₃, -COR⁵, -CH=NOR⁵, -CH₂CONH₂, -CSNHR⁵, -CH=NNHCSNH₂, -CH=NNHCONHNH₂, -C≡CF₃, -CF=CFCF₃, -CF₂-CF₂-CF₃, -CR⁴(CN)₂, -COCF₂CF₂CF₃, -C(CF₃)₃, -C(CN)₃, -CR⁴=C(CN)₂, -1-pyrryl, -C(CN)=C(CN)₂, -C-pyridyl, -COC₆H₅, -COOC₆H₅, -SOCF₃, -SO₂CF₃, -SCF₃, -SO₂CN, -SCOCF₃, -SOR⁵, -S(OR⁵), -SC≡CR⁴, -SO₂R⁵, -SSO₂R⁵, -SR⁵, -SSR⁴, -SO₂CF₂CF₃, -SCF₂CF₃, -S(CF₃)=NSO₂CF₃, -SO₂C₆H₅, -SO₂N(R⁵)₂, -SO₂C(CF₃)₃, -SC(CF₃)₃, -SO(CF₃)=NSO₂CF₃, -S(O)=NCF₃, -S(O)=NR⁵, -S-C=CH₂, -SCOR⁵, -SOC₆H₅, -P(O)C₃F₇, -PO(R⁵)₂, -PO(N(R⁵)₂)₂, -P(N(R⁵)₂)₂, -P(O)R⁵₂, and -PO(OR⁵)₂, or another electron-drawing group, wherein the respective substituent can be in ortho, meta or para position.

In preferred embodiments of compounds of Formula I, **G** is selected from -F, -Cl, -Br, -NO₂, -NR⁴SO₂R⁵, -NHCSNHR⁴, -NR⁴CN, -NR⁴SO₂CF₃, -N=C, -NR⁴COR⁴, -NR⁴COOR⁵, -OSO₂R⁵, -OCF₃, -C=N, -COOR⁴, -CONR⁴R⁵, -COCF₃, -CF₂CF₃, -C≡CR⁴, -COR⁵, -CH₂CONH₂, -CF₃, -C≡CF₃, -CF₂-CF₂-CF₃, -C(CN)=C(CN)₂, -COC₆H₅, -SO₂CF₃, -SCOCF₃, -SO₂R⁵, -SO₂CF₂CF₃, -SO₂C₆H₅, -SO₂N(R⁵) ₂, and -PO(OR⁵)₂, wherein the respective substituent can be in ortho, meta or para position.

In further preferred embodiments of compounds of Formula I, G is selected from -F, -Cl, -Br, -NO₂, -NR⁴SO₂R⁵, -NR⁴COR⁴, -NR⁴COOR⁵, -C≡N, -CONR⁴R⁵, -C≡CR⁴, -COR⁵, -CF₃, -COC₆H₅, -SO₂CF₃, -SO₂R⁵, -SO₂C₆H₅, -SO₂N(R⁵)₂, wherein the respective substituent can be in ortho, meta or para position.

**R¹, R²** and **R³** are independently from each other alkyl or aralkyl.

In preferred embodiments of compounds of Formula I, **R¹, R²** and **R³** are independently from each other substituted or un-substituted alkyl or aralkyl.

In further preferred embodiments of compounds of Formula I, **R¹**, **R²** and **R³** are independently from each other lower un-branched or branched alkyl or aralkyl.

As used in the specification and appended claims, unless specified to the contrary, the term "lower un-branched or branched alkyl" shall have the following meaning: a substituted or unsubstituted, straight or branched chain monovalent or divalent radical consisting substantially of carbon and hydrogen, containing no unsaturation and having from one to eight carbon atoms, e.g. but not limited to methyl, ethyl, n-propyl, n-pentyl, 1,1-dimethylethyl (t-butyl), n-heptyl, and the like.

In preferred embodiments of compounds of Formula I, **R¹, R²** and **R³** are independently from each other aralkyl or lower alkyl whereas one of the three moieties (R¹, R², R³) can be resin-bound.

In preferred embodiments of compounds of Formula I, **R¹**, **R²** and **R³** are independently from each other aralkyl or lower alkyl, whereas at least two moieties of the three moieties (R¹, R², R³) are alkyl.

In preferred embodiments of compounds of Formula I, **R¹** is selected from aralkyl and **R²** and **R³** are methyl.

In further preferred embodiments of compounds of Formula I, **R¹, R²** and **R³** are methyl.

**R⁴** is independently from each other hydrogen or lower un-branched or branched alkyl.

In preferred embodiments of compounds of Formula I, **R⁴** is independently from each other hydrogen or un-branched or branched C₁-C₄ alkyl.

In further preferred embodiments of compounds of Formula I, **R⁴** is independently from each other hydrogen or methyl.

**R⁵** is lower un-branched or branched alkyl.

In preferred embodiments of compounds of Formula I, **R⁵** is un-branched or branched C₁-C₄ alkyl.

In further preferred embodiments of compounds of Formula I, **R⁵** is methyl.

**Q** is hydrogen, lower unbranched or branched alkyl, aryl, heteroaryl, -O-(C₁-C₄Alkyl), -CN, -halo, -SO₂-R⁴ or nitro, wherein respective substituent can be in *ortho, meta* or *para* position or a condensed aryl or heteroaryl.

The term "aryl" as employed herein by itself or as part of another group refers to monocyclic or bicyclic aromatic groups containing from 6 to 12 carbons in the ring portion, preferably 6-10 carbons in the ring portion, such as phenyl, naphthyl or tetrahydronaphthyl.
The term "heteroaryl" as employed herein refers to groups having 5 to 14 ring atoms; 6, 10 or 14 Π (pi) electrons shared in a cyclic array; and containing carbon atoms and 1, 2, 3 or 4 oxygen, nitrogen or sulfur heteroatoms (where examples of heteroaryl groups are: thienyl, benzo[b]thienyl, naphtho[2,3-b]thienyl, thianthrenyl, furyl, pyranyl, isobenzofuranyl, benzoxazolyl, chromenyl, xanthenyl, phenoxathiinyl, 2*H*-pyrrolyl, pyrrolyl, imidazolyl, pyrazolyl, pyridyl, pyrazinyl, pyrimidinyl, pyridazinyl, indolizinyl, isoindolyl, 3*H*-indolyl, indolyl, indazolyl, purinyl, 4*H*-quinolizinyl, isoquinolyl, quinolyl, phthalazinyl, naphthyridinyl, quinazolinyl, cinnolinyl, pteridinyl, 4a*H*-carbazolyl, carbazolyl, carbolinyl, phenanthridinyl, acridinyl, perimidinyl, phenanthrolinyl, phenazinyl, isothiazolyl, phenothiazinyl, isoxazolyl, furazanyl and phenoxazinyl groups).

In preferred embodiments of compounds of Formula I, **Q** is hydrogen, C₁-C₄ alkyl, -O-(C₁-C₄Alkyl), -CN, -fluoro, -chloro, -bromo or nitro, wherein respective substituent can be in ortho, meta or para position.

In further preferred embodiments of compounds of Formula I, **Q** is hydrogen, methyl, -O-(methyl), -CN, -fluoro, -chloro, or nitro, wherein respective substituent can be in ortho, meta or para position.

**U** is a targeting agent.

For the purposes of this invention, the term "targeting agent" shall have the following meaning: The targeting agent is a compound or moiety that targets or directs the radionuclide attached to it to a specific site in a biological system. A targeting agent can be any compound or chemical entity that binds to or accumulates at a target site in a mammalian body, i.e., the compound localizes to a greater extent at the target site than to surrounding tissue.

Preferably the targeting agent **U** is a peptide.

For the purpose of the present invention, the term "peptide" refers to a molecule comprising an amino acid sequence of at least two amino acids.
For the purpose of the present invention the term "amino acid sequence" is defined herein as a polyamide obtainable by polycondensation of at least two amino acids. For the purpose of the present invention the term "amino acid" means any molecule comprising at least one amino group and at least one carboxyl group, but no peptide bond within the molecule. In other words, an amino acid is a molecule that has a carboxylic acid functionality and an amine nitrogen having at least one free hydrogen, preferably in alpha position thereto, but no amide bond in the molecule structure. Thus, a dipeptide having a free amino group at the N-terminus and a free carboxyl group at the C-terminus is not to be considered as a single "amino acid" in the above definition. An amide bond as used herein means any covalent bond having the structure 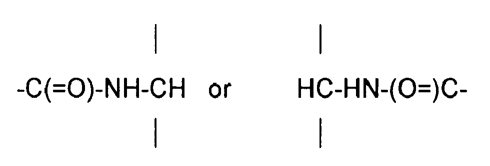 wherein the carbonyl group is provided by one molecule and the NH-group is provided by the other molecule to be joined. The amide bonds between two adjacent amino acid residues which are obtained from such a polycondensation are defined as "peptide bonds". Optionally, the nitrogen atoms of the polyamide backbone (indicated as NH above) may be independently alkylated, e.g. with -C₁-C₆-alkyl, preferably -CH₃.

For the purpose of the specification an amino acid residue is derived from the corresponding amino acid by forming a peptide bond with another amino acid.

For the purpose of the specification an amino acid sequence may comprise naturally occurring and/or artificial amino acid residues, proteinogenic and/or non-proteinogenic amino acid residues. The non-proteinogenic amino acid residues may be further classified as (a) homo analogues of proteinogenic amino acids, (b) β-homo analogues of proteinogenic amino acid residues and (c) further non-proteinogenic amino acid residues.

Accordingly, the amino acid residues are derived from the corresponding amino acids, e.g. from
- proteinogenic amino acids, namely Ala, Arg, Asn, Asp, Cys, Gln, Glu, Gly, His, Ile, Leu, Lys, Met, Phe, Pro, Ser, Thr, Trp, Tyr and Val; or
- non-proteinogenic amino acids, such as
   ○ homo analogues of proteinogenic amino acids wherein the sidechain has been extended by a methylene group, e.g. Homoalanine (Hal), Homoarginine (Har), Homocysteine (Hcy), Homoglutamine (Hgl), Homohistidine (Hhi), Homoisoleucine (Hil), Homoleucine (Hle), Homolysine (Hly), Homomethionine (Hme), Homophenylalanine (Hph), Homoproline (Hpr), Homoserine (Hse), Homothreonine (Hth), Homotryptophane (Htr), Homotyrosine (Hty) and Homovaline (Hva);
   ○ β-homo analogues of proteinogenic amino acids wherein a methylene group has been inserted between the α-carbon and the carboxyl group yielding β-amino acids, e.g. β-Homoalanine (βHal), β-Homoarginine (βHar), β-Homoasparagine (βHas), β-Homocysteine (βHcy), β-Homoglutamine (βHgl), β-Homohistidine (βHhi), β-Homoisoleucine (βHil), β-Homoleucine (βHle), β-Homolysine (βHly), β-Homomethionine (βHme), β-Homophenylalanine (βHph), β-Homoproline (βHpr), β-Homoserine (βHse), β-Homothreonine (βHth), β-Homotryptophane (βHtr), β-Homotyrosine (βHty) and β-Homovaline (βHva);
   ○ further non-proteinogenic amino acids, e.g. α-Aminoadipic acid (Aad), β-Aminoadipic acid (βAad), α-Aminobutyric acid (Abu), α-Aminoisobutyric acid (Aib), β-Alanine (βAla), 4-Aminobutyric acid (4-Abu), 5-Aminovaleric acid (5-Ava), 6-Aminohexanoic acid (6-Ahx), 8-Aminooctanoic acid (8-Aoc), 9-Aminononanoic acid (9-Anc), 10-Aminodecanoic acid (10-Adc), 12-Aminododecanoic acid (12-Ado), α-Aminosuberic acid (Asu), Azetidine-2-carboxylic acid (Aze), β-Cyclohexylalanine (Cha), Citrulline (Cit), Dehydroalanine (Dha), γ-Carboxyglutamic acid (Gla), α-Cyclohexylglycine (Chg), Propargylglycine (Pra), Pyroglutamic acid (Glp), α-tert-Butylglycine (Tle), 4-Benzoylphenylalanine (Bpa), δ-Hydroxylysine (Hyl), 4-Hydroxyproline (Hyp), allo-Isoleucine (alle), Lanthionine (Lan), (1-naphthyl)alanine (1-Nal), (2-naphthyl)alanine (2-Nal), Norleucine (Nle), Norvaline (Nva), Ornithine (Orn), Phenylglycin (Phg), Pipecolic acid (Pip), Sarcosine (Sar), Selenocysteine (Sec), Statine (Sta), β-Thienylalanine (Thi), 1,2,3,4-Tetrahydroisochinoline-3-carboxylic acid (Tic), allo-Threonine (aThr), Thiazolidine-4-carboxylic acid (Thz), γ-Aminobutyric acid (GABA), iso-Cysteine (iso-Cys), Diaminopropionic acid (Dpr), 2,4-Diaminobutyric acid (Dab), 3,4-Diaminobutyric acid (γ,βDab), Biphenylalanine (Bip), Phenylalanine substituted in para-position with -C₁-C₆-alkyl, -halide, -NH₂ or -CO₂H (Phe(4-R) wherein R = -C₁-C₆-alkyl, -halide, -NH₂, or -CO₂H); peptide nucleic acids (PNA, cf. P.E. Nielsen, Acc.Chem.Res. 32, 624-30)
- or their N-alkylated analogues, such as their N-methylated analogues.

Cyclic amino acids may be proteinogenic or non-proteinogenic, such as Pro, Aze, Glp, Hyp, Pip, Tic and Thz.

For further examples and details reference can be made to e.g. J.H. Jones, J. Peptide Sci. 2003, 9, 1-8 which is incorporated herein by reference.

The terms "non-proteinogenic amino acid" and "non-proteinogenic amino acid residue" also encompasses derivatives of proteinogenic amino acids. For example, the sidechain of a proteinogenic amino acid residue may be derivatized thereby rendering the proteinogenic amino acid residue "non-proteinogenic". The same applies to derivatives of the C-terminus and/or the N-terminus of a proteinogenic amino acid residue terminating the amino acid sequence.

For the purpose of the specification a proteinogenic amino acid residue is derived from a proteinogenic amino acid selected from the group consisting of Ala, Arg, Asn, Asp, Cys, Gln, Glu, Gly, His, Ile, Leu, Lys, Met, Phe, Pro, Ser, Thr, Trp, Tyr and Val either in L- or D-configuration; the second chiral center in Thr and Ile may have either R- or S-configuration. Therefore, for example, any posttranslational modification of an amino acid sequence, such as N-alkylation, which might naturally occur renders the corresponding modified amino acid residue "non-proteinogenic", although in nature said amino acid residue is incorporated in a protein.

Examples for peptides as targeting agent U are, but are not limited to, somatostatin and derivatives thereof and related peptides, neuropeptide Y and derivatives thereof and related peptides, bombesin and derivatives thereof and related peptides, gastrin, gastrin releasing peptide derivatives thereof and related peptides, epidermal growth factor (EGF of various origin), insulin growth factor (IGF) and IGF-1, LHRH agonists and antagonists, transforming growth factors, particularly TGF-□; angiotensin; cholecystokinin (CCK) and analogs; neurotensin and analogs, thyrotropin releasing hormone, prolactin, tumor necrosis factor, IL-1, IL-2, IL-4 or IL-6, interferons, VIP and related peptides, PACAP and related peptides. Such peptides comprise from 4 to 100 amino acids, wherein the amino acids are selected from natural and non-natural amino acids and also comprise modified natural and non-natural amino acids.

In other preferred embodiments **U** is selected to be an oligonucleotide.

For the purposes of this invention, the term "oligonucleotide" shall have the following meaning: short sequences of nucleotides, typically with twenty or fewer bases. Examples are, but are not limited to, molecules named and cited in the book: "The aptamers handbook. Functional oligonuclides and their application" by Svenn Klussmann, Wiley-VCH, 2006. An example for such an oligonucleotide is TTA1 (J. Nucl. Med., 2006, April, 47(4), 668-78).
For the purpose of this invention, the term "aptamer" refers to an oligonucleotide, comprising from 4 to 100 nucleotides, wherein at least two single nucleotides are connected to each other via a phosphodiester linkage. Said aptamers have the ability to bind specifically to a target molecule (see e.g. M Famulok, G Mayer, Aptamers as Tools in Molecular Biology and Immunology, In: Combinatorial Chemistry in Biology, Current Topics in Microbiology and Immunology (M Famulok, CH Wong, EL Winnacker, Eds.), Springer Verlag Heidelberg, 1999, Vol. 243, 123-136). There are many ways known to the skilled person of how to generate such aptamers that have specificity for a certain target molecule. An example is given in WO 01/09390, the disclosure of which is hereby incorporated by reference. Said aptamers may comprise substituted or non-substituted natural and non-natural nucleotides. Aptamers can be synthesized in vitro using e.g. an automated synthesizer. Aptamers according to the present invention can be stabilized against nuclease degradation e.g. by the substitution of the 2'-OH group versus a 2'-fluoro substituent of the ribose backbone of pyrimidine and versus 2'-O-methyl substituents in the purine nucleic acids. In addition, the 3' end of an aptamer can be protected against exonuclease degradation by inverting the 3' nucleotide to form a new 5'-OH group, with a 3' to 3' linkage to a penultimate base.

For the purpose of this invention, the term "nucleotide" refers to molecules comprising a nitrogen-containing base, a 5-carbon sugar, and one or more phosphate groups. Examples of said base comprise, but are not limited to, adenine, guanine, cytosine, uracil, and thymine. Also non-natural, substituted or non-substituted bases are included. Examples of 5-carbon sugar comprise, but are not limited to, D-ribose, and D-2-desoxyribose. Also other natural and non-natural, substituted or non-substituted 5-carbon sugars are included. Nucleotides as used in this invention may comprise from one to three phosphates.

Small molecules effective for targeting certain sites in a biological system can also be used as the targeting agent **U**.

For the purposes of this invention, the term "small molecule" shall have the following meaning: a small molecule is a compound that has a molecular mass of 200 to 800 and that contains a primary and/or secondary amine to which compounds of Formula I and II are coupled via L. Such targeting moieties are known in the art, so are methods for preparing them.

Preferred as targeting agents **U** are peptides comprising from 4 to 100 amino acids or oligonucleotides comprising from 4 to 100 nucleotides or peptidomimetics.

In preferred embodiments of compounds of Formula I, **U** is -NR⁴-peptide, -(CH₂)ₙ-peptide, -small-molecule or -(CH₂)ₙ-small molecule, or -NR⁴-oligonucleotide or -(CH₂)ₙ-oligonucleotide and n is an integer between 1 and 6.

In further preferred embodiments of compounds of Formula I, **U** is -NR⁴-peptide, or-(CH₂)ₙ-peptide, and n is an integer between 1 and 6.

In other preferred embodiments of compounds of Formula I, **U** is , -NR⁴-oligonucleotide or -(CH₂)ₙ-oligonucleotide and n is an integer between 1 and 6.

In preferred embodiments of compounds of Formula I, **U** is small-molecule or-(CH₂)ₙ-small molecule, and n is an integer between 1 and 6.

**X**⁻ is CF₃S(O)₂O⁻, C₄F₉S(O)₂O⁻, iodide anion, bromide anion, chloride anion, perchlorate anion (ClO₄⁻), phosphate anion or other salts of inorganic or organic acids.

In preferred embodiments of compounds of Formula **I**, **X⁻** is CF₃S(O)₂O⁻ or C₄F₉S(O)₂O⁻.

In further preferred embodiments of compounds of Formula I, **X⁻** is CF₃S(O)₂O⁻.

The term "salts of inorganic or organic acids" as employed herein refers to mineral acids, including but not limited to: acids such as carbonic, nitric or sulphuric acid or to appropriate organic acids which includes but not limited to: acids such as aliphatic, cycloaliphatic, aromatic, araliphatic, heterocyclic, carboxylic and sulphonic acids, examples of which are formic, acetic, trifluoracetic, propionic, succinic, glycolic, gluconic, lactic, malic, fumaric, pyruvic, benzoic, anthranilic, mesylic, fumaric, salicylic, phenylacetic, mandelic, embonic, methansulfonic, ethanesulfonic, benzenesulfonic, phantothenic, toluenesulfonic and sulfanilic acid.

It has been found that compounds according to Formula I can be ¹⁸F-labeled surprisingly in a one-step radiofluorination reaction in order to arrive at compounds according to Formula II.

In a second aspect the present invention is directed to compounds of Formula II 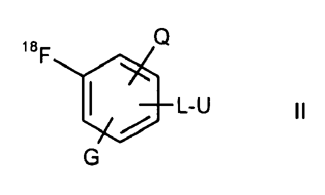 wherein:
**L** is a bond, -CO-, -SO₂-, -(CH₂)_{d}-CO-, -SO-, or -C≡C-CO-, wherein the respective substituent can be in ortho, meta or para-position and d is an integer between 1 and 6.

In preferred embodiments of compounds of Formula II, L is -CO-, -SO₂-, or -C≡C-CO-, wherein the respective substituent can be in meta or para position.

In further preferred embodiments of compounds of Formula II, **L** is -CO-, or -SO₂, wherein the respective substituent can be in meta or para position.

**G** is selected from -F, -Cl, -Br, -I, -NO, -NO₂, -NR⁴COCF₃, -NR⁴SO₂CF₃, -N(CF₃)₂, -NHCSNHR⁴, -N(SO₂R⁵)₂, -N(O)=NCONH₂, -NR⁴CN, -NHCSR⁵, -N≡C, -N=C(CF₃)₂, -N=NCF₃, -N=NCN, -NR⁴COR⁴, -NR⁴COOR⁵, -OSO₂CF₃ , -OSO₂C₆H₅, -OCOR⁵, -ONO₂, -OSO₂R⁵, -O-C=CH₂, -OCF₂CF₃, -OCOCF₃, -OCN, -OCF₃, -C=N, -C(NO₂)₃, -COOR⁴, -CONR⁴R⁵, -CSNH₂, -CH=NOR⁴, -CH₂SO₂R⁴, -COCF₃, -CF₃, -CF₂Cl-CBr₃, -CCIF₂, -CCI₃, -CF₂CF₃, -C≡CR⁴, -CH=NSO₂CF₃, -CH₂CF₃, -COR⁵, -CH=NOR⁵, -CH₂CONH₂, CSNHR⁵, -CH=NNHCSNH₂, -CH=NNHCONHNH₂, -C≡CF₃, -CF=CFCF₃, -CF₂-CF₂-CF₃, -CR⁴(CN)₂, -COCF₂CF₂CF₃, -C(CF₃)₃, -C(CN)₃, -CR⁴=C(CN)₂, -1-pyrryl, -C(CN)=C(CN)₂, -C-pyridyl, -COC₆H₅, -COOC₆H₅, -SOCF₃, -SO₂CF₃, -SCF₃, -SO₂CN, -SCOCF₃, -SOR⁵, -S(OR⁵), -SC≡CR⁴, -SO₂R⁵, -SSO₂R⁵, -SR⁵, -SSR⁴, -SO₂CF₂CF₃, -SCF₂CF₃, -S(CF₃)=NSO₂CF₃, -SO₂C₆H₅, -SO₂N(R⁵)₂, -SO₂C(CF₃)₃, -SC(CF₃)₃, -SO(CF₃)=NSO₂CF₃, -S(O)=NCF₃, -S(O)=NR⁵, -S-C=CH₂, -SCOR⁵, -SOC₆H₅, -P(O)C₃F₇, -PO(R⁵)₂, -PO(N(R⁵)₂)₂, -P(N(R⁵)₂)₂, -P(O)R⁵₂, and -PO(OR⁵)₂, or another electron-drawing group, wherein the respective substituent can be in ortho, meta or para position.

In a preferred embodiment of compounds of Formula II, **G** is selected from -F, -Cl, -Br, -NO₂, -NR⁴SO₂R⁵, -NHCSNHR⁴, -NR⁴CN, -NR⁴SO₂CF₃, -N≡C, -NR⁴COR⁴, -NR⁴COOR⁵, -OSO₂R⁵, -OCF₃, -C=N, -COOR⁴, -CONR⁴R⁵, -COCF₃, -CF₂CF₃, -C≡CR⁴, -COR⁵, -CH₂CONH₂, -CF₃, -C≡CF₃, -CF₂-CF₂-CF₃, -C(CN)=C(CN)₂, -COC₆H₅, -SO₂CF₃, -SCOCF₃, -SO₂R⁵, -SO₂CF₂CF₃, -SO₂C₆H₅, -SO₂N(R⁵) ₂, and -PO(OR⁵)₂, wherein the respective substituent can be in ortho, meta or para position.

In further preferred embodiments of compounds of Formula II, **G** is selected from -F, -Cl, -Br, -NO₂, -NR⁴SO₂R⁵, -NR⁴COR⁴, -NR⁴COOR⁵, -C=N, -CONR⁴R⁵, -C≡CR⁴, -COR⁵, -CF₃, -COC₆H₅, -SO₂CF₃, -SO₂R⁵, -SO₂C₆H₅, -SO₂N(R⁵)₂, wherein the respective substituent can be in ortho, meta or para position.

**R⁴** is independently from each other hydrogen or lower un-branched or branched alkyl.

In a preferred embodiment of compounds of Formula II, **R⁴** is independently from each other hydrogen or un-branched or branched C₁-C₄ alkyl.

In further preferred embodiments of compounds of Formula II, **R⁴** is independently from each other hydrogen or methyl.

**R⁵** is lower un-branched or branched alkyl.

In preferred embodiments of compounds of Formula II, **R⁵** is un-branched or branched C₁-C₄ alkyl.

In further preferred embodiments of compounds of Formula II, **R⁵** is methyl.

**Q** is hydrogen, lower un-branched or branched alkyl, aryl, heteroaryl, -O-(C₁-C₄Alkyl), -CN, -halo, -SO₂-R⁴ or nitro, wherein respective substituent can be in *ortho, meta* or *para* position or a condensed aryl or heteroaryl.

In preferred embodiments of compounds of Formula II, **Q** is hydrogen, C₁-C₄ alkyl, -O-(C₁-C₄Alkyl), -CN, -fluoro, -chloro, -bromo or nitro, wherein respective substituent can be in ortho, meta or para position.

In further preferred embodiments of compounds of Formula II, **Q** is hydrogen, methyl, -O-(methyl), -CN, -fluoro, -chloro, or nitro, wherein respective substituent can be in ortho, meta or para position.

**U** is a targeting agent.

Preferred as targeting agents U are peptides comprising from 4 to 100 amino acids or oligonucleotides comprising from 4 to 100 nucleotides or peptidomimetics.

In preferred embodiments of compounds of Formula II, **U** is -NR⁴-peptide, -(CH₂)ₙ- peptide, -small-molecule or -(CH₂)ₙ-small molecule, -NR⁴-oligonucleotide or -(CH₂)ₙ- oligonucleotide and n is an integer between 1 and 6.

In further preferred embodiments of compounds of Formula II, **U** is -NR⁴-peptide, or -(CH₂)ₙ-peptide, and n is an integer between 1 and 6.

In other preferred embodiments of compounds of Formula II, **U** is , -NR⁴-oligonucleotide or -(CH₂)ₙ-oligonucleotide and n is an integer between 1 and 6.

In preferred embodiments of compounds of Formula II, **U** is -small-molecule or -(CH₂)ₙ- small molecule, and n is an integer between 1 and 6.

If a chiral center or another form of an isomeric center is present in a compound according to Formula I or II of the present invention, all forms of such isomer, including enantiomers and diastereoisomers, are intended to be covered herein. Compounds containing a chiral center may be used as racemic mixture or as an enantiomerically enriched mixture or the racemic mixture may be separated using well-known techniques and an individual enantiomer maybe used alone. In casesin which compounds have unsaturated carbon-carbon bonds double bonds, both the cis-isomer and trans-isomers are within the scope of this invention. In cases wherein compounds may exist in tautomeric forms, such as keto-enol tautomers, each tautomeric form is contemplated as being included within this invention whether existing in equilibrium or predominantly in one form.

Unless otherwise specified, when referring, to the compounds, of formula I per se as well as to any pharmaceutical composition thereof the present invention includes all of the hydrates, solvates, complexes, and prodrugs of the compounds of the invention. Prodrugs are any covalently bonded compounds, which releases the active parent pharmaceutical according to formula II.

In a further aspect the present invention is directed to a method of preparing a compound of Formula II characterized by reacting a compound of Formula I with an appropriate radiofluorination agent. The reagents, solvents and conditions which can be used for this radiofluorination are common and well-known to the skilled person in the field. See e.g. J. Fluorine Chem. 27 (1985) 117-191.

In a preferred method of preparing a compound of Formula II, the step of radiofluorination of a compound of Formula I is carried out at a temperature at or below 90°C.

In a preferred method of preparing a compound of Formula II, the step of radiofluorination of a compound of Formula I is carried out at a temperature selected from a range from 10°C to 90°C.

In a preferred method of preparing a compound of Formula II, the step of radiofluorination of a compound of Formula I is carried out at a temperature selected from a range from 10°C to 70°C.

In a preferred method of preparing a compound of Formula II, the step of radiofluorination of a compound of Formula I is carried out at a temperature selected from a range from 30°C to 60°C.

In a preferred method of preparing a compound of Formula II, the step of radiofluorination of a compound of Formula I is carried out at a temperature selected from a range from 45 to 55 °C.

In a preferred method of preparing a compound of Formula II, the step of radiofluorination of a compound of Formula I is carried out at a temperature at 50°C.

In the following two schematic examples are given of how to prepare a compound according to Formula II using a compound according to Formula I. The methods presented as schemes below are in principle suitable to generate compounds over the whole breadth of Formula II using compounds over the whole breadth of Formula I. The examples presented below are given merely to illustrate a way of labeling a compound according to Formula I to arrive at a compound according to Formula II and is not to be understood as to limit the invention to the methods exemplified herein.

Scheme 3 depicts an example of a synthetic route for forming a trimethylammonium substituted aromatic moiety containing peptide of Formula I and subsequent direct radiolabeling towards the corresponding ¹⁸F-labeled compound of formula II.
The synthesis starts with commercially available 2-Fluoro-5-formyl-benzonitrile (**3**) which is converted to the corresponding acid (**4**) by known oxidation methods e.g. use of chromium reagents, manganese reagents or other typical reagents which are well-known to experts and which can be taken from, but are not limited to, the methods described and cited in the book: "Modern oxidation methods" by Jan-Erling Bäckvall, Wiley-VCH, 2004. A useful method is e.g. the oxidation with sodium chlorit in phosphate-buffered tert-butanol solution.

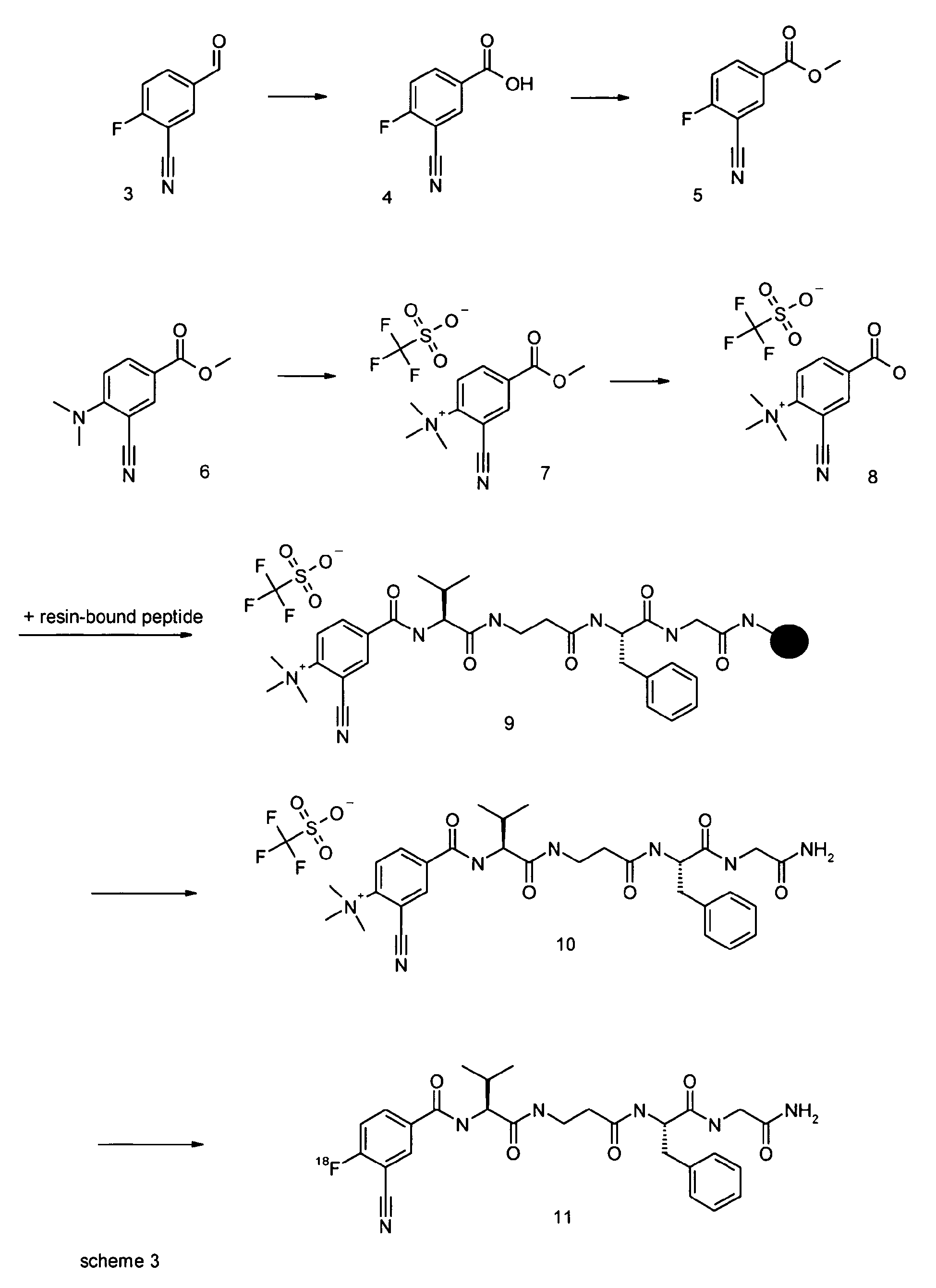

Acid (**4**) can be converted to the corresponding methyl ester with methanol and acetyl chloride (Helv. Chim. Acta (2005); 88; 7; 1630 - 1657). But the preparation of other alkyl esters (e.g. ethyl ester) and other esterfication methods including variants under basic conditions are also possible and useful and are well known to experts. The displacement of fluoride (**5**) with dimethylamine or dimethylamine hydrochloride by nucleophilic aromatic substitution reaction can be carried out e.g. in a suspension of DMSO and potassium carbonate (e.g. Bioorg. Med. Chem. Lett.; 10; 23; 2000; 2603 - 2606). Useful other solvents can be selected from, but are not limited to, acetonitril and DMF. The quarternisation of aniline (**6**) with methyl iodide, methyl triflate or other alkylating agents is carried out in boiling dichlormethane with normal or increased pressure (e.g. 1-10 bar). Other useful solvents for this reaction can be selected from, but are not limited to, acetone and dichloroethane. The crude product can be purified by reverse-phase column chromatography. The methyl ester (**7**) can be cleaved in boiling trifluoro acetic acid and water (Bioorg. Med. Chem. 2003, 11, 4189-4206) or under other acidic conditions. The acid (**8**) is coupled by solid phase synthesis (as shown in scheme 3) to solid-phase bound peptide to obtain amide (**9**) (or esters) by methods which are well known to experts. Typical condensating agents for those kinds of couplings are diisopropylcarbodiimid or dicyclohexylcarbodiimid, but also other condensating agents (see e.g. Chan and White ("Fmoc Solid Phase Peptide Synthesis - A Practical Approach"), are possible. The resin-bound peptide is then cleaved from the resin by acidification to obtain liberated peptide (**10**). The cleavage is also possible by other appropriate methods which are very much dependent on the kind of linker. The methods of peptide cleavage from resin are very well known to experts and described in literature (e.g. Chan and White - "Fmoc Solid Phase Peptide Synthesis - A Practical Approach"). The purified peptide (**10**) is converted at 70°C ± 45°C with [¹⁸F] potassium fluoride, potassium carbonate and Kryptofix (4,7,13,16,21,24-Hexaoxa-1,10-diaza-bicyclo[8.8.8]hexacosane) in dimethylsuphoxide to obtain the desired ¹⁸F-labeled peptide (**11**). The reagents and solvents which are used for this radiofluorination are common, well-known to experts since many years and described in many publications (e.g. J. Fluorine Chem., 27 (1985) 117-191). It was surprisingly found that the radiofluorination does take place and that the temperature can be decreased to <90°C so that the peptide is not harmed or decomposed. The preferred temperature range for conducting the radioflurination of compounds according to Formula I is from 10°C to 90°C. More preferred is the temperature range of 10°C to 70°C. Even more preferred is the range of temperature from 35°C to 65°C. Further preferred is a range of temperature from 45°C to 55°C. Most preferred is a temperature of 50°C for carrying out the radiofluorination of compounds according to Formula I to arrive at compounds according to Formula II.

Another example of a trimethyl ammonium derivative was prepared as shown in scheme 4: Commercially available sulfonylchloride **12** was coupled with the secondary amine sarcosin methyl ester to the sulfonamide **13** in dichlormethane and diisopropyl ethyl amine to scavenge hydrogen chloride. Other useful solvents for this reaction can be, but are not limited to, DMF, THF, dioxane, dichloroethane, diethylether and tert butyl ether. Other useful bases can be, but are not limited to, trimethylamine, N-methyl morpholine, NMP and sodium hydrogen carbonate.

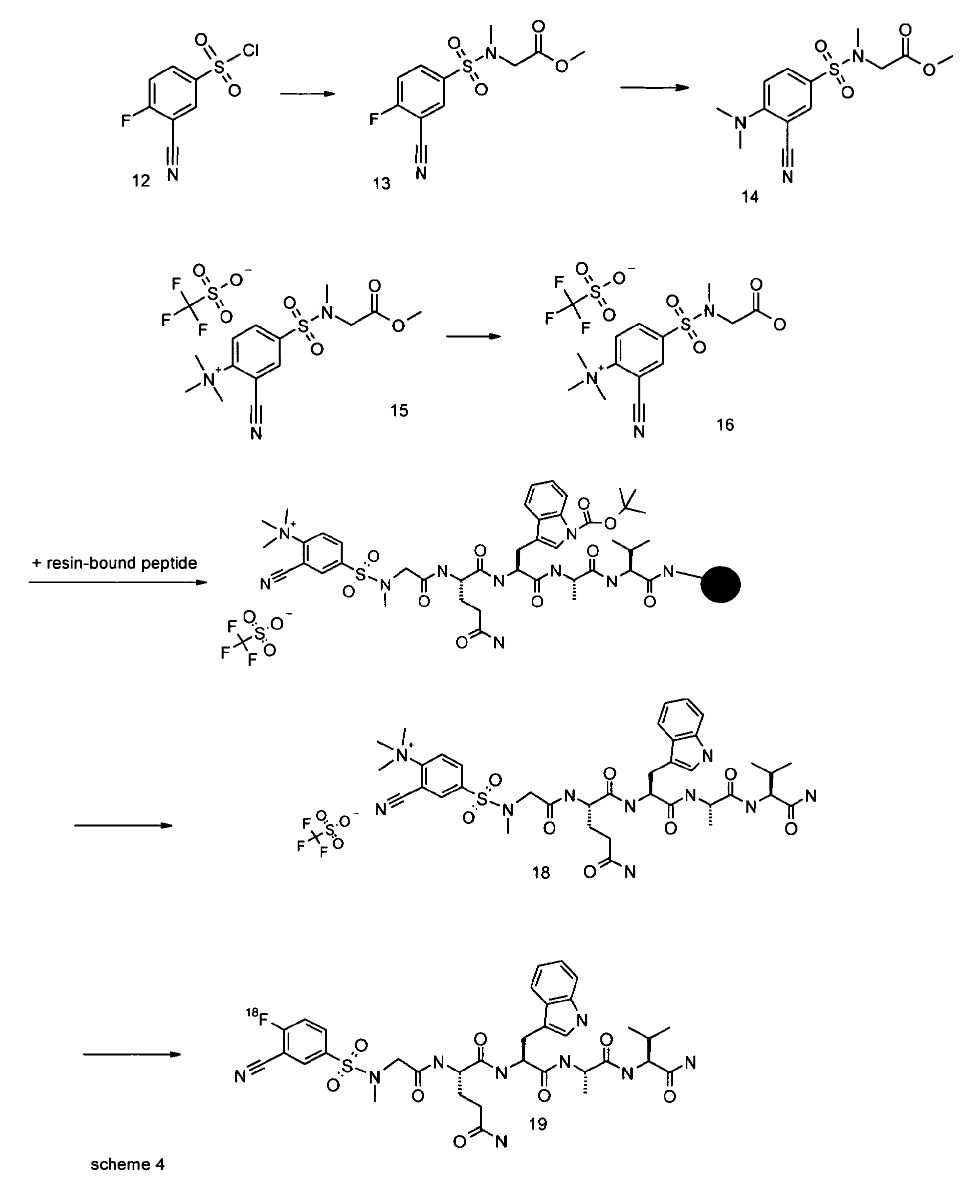

Sulfonamide **13** was then treated in an aromatic nucleophilic substitution reaction with dimethylammonium hydrochloride and potassium carbonate in dimethylsulphoxide to obtain dimethylaniline **14.** The aniline derivative **14** was quarternized with methyltriflate or methyl iodide in dichloromethane or dichloroethane towards ammonium salt **15.** The resulting ester **15** was cleaved under acidic conditions, e.g. in boiling trifluoro acetic acid and water (Bioorg. Med. Chem. 2003, 11, 4189-4206). The acid **16** was condensed to the resin-bound peptide by methods which are well known to experts. Typical condensating agents for those kinds of couplings are diisopropylcarbodiimid or dicyclohexylcarbodiimid, but also other condensating agents are possible and described. Examples are given in, but are not limited to, the methods described and cited in the book: Chan and White - "Fmoc Solid Phase Peptide Synthesis - A Practical Approach". The resin-bound trimethylammonium peptide **17** is then cleaved from the resin by acidification to obtain liberated peptide **(18).** The cleavage is also possible by other appropriate methods which are very much dependent on the kind of linker. The methods of peptide cleavage from resin are very well known to experts. Examples are given in, but are not limited to, the methods described and cited in the book: Chan and White - "Fmoc Solid Phase Peptide Synthesis - A Practical Approach". The purified peptide (**18**) is converted at 70°C ± 45°C with [¹⁸F] potassium fluoride, potassium carbonate and Kryptofix (4,7,13,16,21,24-Hexaoxa-1,10-diaza-bicyclo[8.8.8]hexacosane) in dimethylsufoxide to obtain the desired ¹⁸F-labeled peptide **(19).** The reagents and solvents which are used for this radiofluorination are common, well-known to experts and are described in many publications (e.g. J. Fluorine Chem., 27 (1985) 117-191). Beside potassium carbonate as base also tetraalkyl ammonium carbonate is possible. It was surprisingly found that the radiofluorination does take place at the given mild temperatures so that the peptide is not harmed or decomposed. The preferred temperature range for conducting the radioflurination of compounds according to Formula I is from 10°C to 90°C. More preferred is the temperature range of 10°C to 70°C. Even more preferred is the range of temperature from 35°C to 65°C. Further preferred is a range of temperature from 45°C to 55°C. Most preferred is a temperature of 50°C for carrying out the radiofluorination of compounds according to Formula I to arrive at compounds according to Formula II.

The invention also provides compositions comprising compound of Formula I or II and a pharmaceutically acceptable carrier or diluent. Pharmaceutically acceptable carrier or diluent may include any and all solvents, dispersion media, antibacterial and antifungal agents, isotonic agents, enzyme inhibitors, transfer ligands such as glucoheptonate, tartrate, citrate, or mannitol, and the like. Such compositions may be formulated as sterile, pyrogen-free, parenterally acceptable aqueous solution which may optionally be supplied in lyophilized form. The compositions of the invention may be provided as components of kits which may include buffers, additional vials, instructions for use, and the like.

In a further aspect the present invention provides a kit comprising a sealed vial containing a predetermined quantity of a compound according to Formula I.

In another aspect of the invention compounds according to Formula II are provided for use as medicament.

In another aspect of the invention compounds according to Formula II are provided for use as diagnostic imaging agent, preferably as imaging agent for PET applications.

In a further aspect of the invention, compounds according to Formula I are provided for the use in manufacturing of a medicament.

In a further aspect of the invention, compounds according to Formula II are provided for the use in manufacturing of a medicament.

In a further aspect of the invention, compounds according to Formula I are provided for the use in manufacturing of a diagnostic imaging agent.

In a further aspect of the invention, compounds according to Formula II are provided for the use in manufacturing of a diagnostic imaging agent.

The radioactively labeled compounds according to Formula II provided by the invention may be administered intravenously in any pharmaceutically acceptable carrier, e.g. conventional medium such as an aqueous saline medium, or in blood plasma medium, as a pharmaceutical composition for intravenous injection. Such medium may also contain conventional pharmaceutical materials such as, for example, pharmaceutically acceptable salts to adjust the osmotic pressure, buffers, preservatives and the like. Among the preferred media are normal saline and plasma. Suitable pharmaceutical acceptable carriers are known to the person skilled in the art. In this regard reference can be made to e.g. Remington's Practice of Pharmacy, 11th ed.

The concentration of the compound according to Formula II and the pharmaceutically acceptable carrier, for example, in an aqueous medium, varies with the particular field of use. A sufficient amount is present in the pharmaceutically acceptable carrier when satisfactory visualization of the imaging target (e.g. a tumor) is achievable.

In accordance with the invention, the radiolabeled compounds according to Formula II either as a neutral composition or as a salt with a pharmaceutically acceptable counterion are administered in a single unit injectable dose. Any of the common carriers known to those with skill in the art, such as sterile saline solution or plasma, can be utilized after radiolabelling for preparing the injectable solution to diagnostically image various organs, tumors and the like in accordance with the invention. Generally, the unit dose to be administered for a diagnostic agent has a radioactivity of about 0.1 mCi to about 100 mCi, preferably 1 mCi to 20 mCi. For a radiotherapeutic agent, the radioactivity of the therapeutic unit dose is about 10 mCi to 700 mCi, preferably 50 mCi to 400 mCi. The solution to be injected at unit dosage is from about 0.01 ml to about 30 ml. For diagnostic purposes after intravenous administration, imaging of the organ or tumor in vivo can take place in a matter of a few minutes. However, imaging takes place, if desired, in hours or even longer, after injecting into patients. In most instances, a sufficient amount of the administered dose will accumulate in the area to be imaged within about 0.1 of an hour to permit the taking of scintigraphic images. Any conventional method of scintigraphic imaging for diagnostic purposes can be utilized in accordance with this invention.

In a further aspect of the invention the proposed methods can also be used to introduce other halogens or radioactive isotopes thereof, e.g. ¹⁹F , ⁷⁵Br, ⁷⁶Br, ¹²³I, ¹³¹I or ^{34m}Cl.

### Examples:

### Example 1

### a) 2-Chloro-4-dimethylamino-benzoic acid methyl ester (1a)

To a stirred solution of 4,00g (20.6 mmol) 2-chloro-4-fluoro-benzoic acid methyl ester (Apollo) and 60 ml dimethylsulphoxid are added 2.03 g (24,7 mmol) dimethylamine hydrochloride and 5,97 g (43.2 mmol) potassium carbonate. The reaction mixture is stirred over night and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude product **1a** is obtained in 99% yield (4,36 g, 20.4 mmol) and is used for the next step without purification.
MS-ESI: 213/215 (M⁺ +1, 64/48).

| Elementary analysis: | | | |
|---|---|---|---|
| Calculated: | C 56.21% | H 5.66% | N 6.56% |
| Determined: | C 56.39% | H 5,67% | N 6.54% |

### b) Synthesis of (3-chloro-4-methoxycarbonyl-phenyl)-trimethyl-ammonium trifluoromethanesulfonate (1b)

To a stirred solution of 4.49 g (21.0 mmol) **1a** and 75 ml dichloromethane are added 34,5 g (210 mmol) methyltriflate (Aldrich) drop wisely. The reaction mixture is stirred for 2 days at room temperature. 17 g (10 mmol) methyltriflate (Aldrich) are added and the reaction mixture is stirred at 40°C for 20 h. The reaction mixture is cooled to 20°C and diethylether is added. The desired compound precipitates and the solvent is decanted and the solid is washed extensively (ten times) with large amounts of diethylether. The solid is dried by use of oil pump vacuum and purified by (C-18) RP-column chromatography (acetonitril/water - gradient 1:99 to 80:20). The desired compound **1b** is obtained in 86% yield (6,86 g, 18,1 mmol).
MS-ESI: 228/230 (M⁺ , 81),

| Elementary analysis: | | | | |
|---|---|---|---|---|
| Calculated: | C 38.15% | H 4.00% | F 15.09% | N 3.71% |
| Determined: | C 38.18% | H 4.02% | F 15.04% | N 3.70% |

### c) Synthesis of (4-carboxy-3-chloro-phenyl)-trimethyl-ammonium trifluoromethanesulfonate (1c).

A solution of 0.5 g (1.32 mmol) **1b,** 12 ml dest. water and 12 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude **1c** is obtained in 98% yield (471 mg, 1.3 mmol) and crude compound **1c** is used for the next step without purification.
MS-ESI: 214/216 (M⁺ , 89/56).

| Elementary analysis: | | | | |
|---|---|---|---|---|
| Calculated: | C 36.32% | H 3.60% | F 15.67% | N 3.85% |
| Determined: | C 36.37% | H 3.63% | F 15.61% | N 3,83% |

### d) Synthesis of (4-Trimethylammonium-2-chloro-benzoyl)-Trp-Ala-Val-Leu-NH₂- triflate salt (1d).

To a stirred solution of 72.8 mg (0.2 mmol) **1c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Trp(Boc)-Ala-Val-Leu-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 69,4 mg (0.2 mmol) **1c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water : acetonitril gradient and 0.1% trifluoro acetic acid as co-solvent to obtain the desired product (**1d**) in 30.0 % yield - 16.2 mg (0.0195 mmol).
MS-ESI: 683/685 (M⁺, 39 / 26)

### e) Synthesis of [¹⁸F]-(4-Fluoro-2-chloro-benzoyl)-Trp-Ala-Val-Leu-NH₂ (1e).

To a Wheaton vial (5ml) charged with Kryptofix 222 (5mg), potassium carbonate (1mg in 500µl water) and MeCN (1.5ml) the fluorine containing water (316MBq, 33µl) are added. The solvent is removed by heating at 120°C for 10 mins under a stream of nitrogen. Anhydrous MeCN (1ml) is added and evaporated as before. This step is repeated again. A solution of **1d** (2mg) in anhydrous DMSO (300µl) is added. After heating at 80°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **1e** is confirmed by co-injection with the cold F-19 fluoro standard on the Econsphere analytical HPLC.

### Example 2

### a) Synthesis of 3-Cyano-4-fluoro-benzoic acid (2a).

To a stirred solution of 15.0 g (97,6 mmol) 2-fluoro-5-formyl-benzonitrile (Aldrich), 150 ml dest. water and 630 ml t-butanol are added 40.8 g (361 mmol) sodium chlorite and 35.9 g (230 mmol) sodium hydrogen phosphate dihydrate. The reaction mixture is stirred over night and poured into a diluted aqueous hydrogen chloride solution (pH=3.5). The pH value is readjusted to pH=3.5 by aqueous hydrogen chloride. The aqueous solution is extracted trice with dichloromethane/isopropanol (10:1). The combined organic phases are dried (sodium sulphate) and concentrated. The residue is purified by extraction with sodium hydrogen carbonate solution and dichloromethane, acidification with aqueous solution and subsequent filtering. The solid crude product **2a** is obtained in 90% yield (14.5 g, 87.8 mmol) and is used for the next step without purification.
MS-ESI: 166 (M⁺ +1, 77),

| Elementary analysis: | | | | |
|---|---|---|---|---|
| Calculated: | C 58.19% | H 2.44% | F 11.51% | N 8.48% |
| Determined: | C 58.81% | H 2.42% | F 11.41% | N 8,47% |

### b) Synthesis of 3-Cyano-4-fluoro-benzoic acid methyl ester (2b).

To a stirred suspension of 16.0 g (96,9 mmol) **2a** and 161 ml methanol are added 30.4 g (387,6 mmol) acetyl chloride drop wisely at 0°C. The reaction mixture is stirred over night, filtered and concentrated. The residue is diluted with dichloromethane, washed with diluted sodium hydrogen carbonate solution, dried with sodium sulphate and concentrated. The residue is purified by column chromatography (hexane : ethylacetate). The desired product **2b** is obtained in 78,1% yield (13.5 g; 75.7 mmol)
MS-ESI: 180 (M⁺ +1, 57),

| Elementary analysis: | | | | |
|---|---|---|---|---|
| Calculated: | C 60.34% | H 3.38% | F 10.60% | N 7.82% |
| Determined: | C 60.51% | H 3.39% | F 10.57% | N 7,80% |

### c) Synthesis of 3-Cyano-4-dimethylamino-benzoic acid methyl ester (2c).

To a stirred solution of 24.0 g (134 mmol) **2b** and 240 ml dimethylsulphoxid are added 13.2 g (161 mmol) dimethylamine hydrochloride and 38,9 g (281 mmol) potassium carbonate. The reaction mixture is stirred over night and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude product **2c** is obtained in 94% yield (25,7 g, 126 mmol) and is used for the next step without purification.
MS-ESI: 205 (M⁺ +1, 59),

| Elementary analysis: | | | |
|---|---|---|---|
| Calculated: | C 64.69% | H 5.92% | N 13.72% |
| Determined: | C 64.79% | H 5,95% | N 13.69% |

### d) Synthesis of (2-Cyano-4-methoxycarbonyl-phenyl)-trimethyl-ammonium trifluoromethanesulfonate (2d).

To a stirred solution of 6.16 g (30.2 mmol) **2c** and 110 ml dichloromethane are added 50.0 g (302 mmol) methyltriflate (Aldrich) drop wisely. The reaction mixture is stirred over night and diethylether is added. After evaporation of one third of the solvent volume the desired compound precipitates and the rest of the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **2d** is obtained in 69 % yield (20.8 mmol, 7.68 g).
MS-ESI: 219 (M⁺, 100),

| Elementary analysis: | | | | |
|---|---|---|---|---|
| Calculated: | C 42.39% | H 4.10% | F 15.47% | N 7.61% |
| Determined: | C 42.42% | H 4.12% | F 15.41% | N 7.59% |

### e) Synthesis of Trifluoro-methanesulfonate(4-carboxy-2-cyano-phenyl)-trimethylammonium; (2e).

A solution of 4,01 g (10.9 mmol) **2d,** 95 ml dest. water and 95 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **2e** is obtained in 93% yield (3.59 g, 10.1 mmol) and crude compound **2e** is used for the next step without purification.
MS-ESI: 205 (M⁺, 100),

| Elementary analysis: | | | | |
|---|---|---|---|---|
| Calculated: | C 40.68% | H 3.70% | F 16.09% | N 7.91% |
| Determined: | C 40.72% | H 3.71% | F 16.06% | N 7,91% |

### f) Synthesis of peptide

(4-Trimethylammonium-3-cyano-benzoyl)-Arg-Ala-His(π-Me)-Leu-NH₂ - triflate salt, (2f).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Arg(Pbf)-Ala-His(π-Me)-Leu-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water : acetonitril gradient and 0.1% trifluoro acetic acid as co-solvent to obtain the desired product **(2f)** in 27,0 % yield - 14,9 mg (0.0176 mmol).
MS-ESI: 698 (M⁺, 100).

### g) Synthesis of

[¹⁸F]-(4-Fluoro-3-cyano-benzoyl)-Arg-Ala-His(π-Me)-Leu-NH₂ (2g).

To a Wheaton vial (5ml) charged with Kryptofix 222 (5mg), potassium carbonate (1mg in 500µl water) and MeCN (1.5ml) the fluorine containing water (356 MBq, 38 µl) are added. The solvent is removed by heating at 120°C for 10 mins under a stream of nitrogen. Anhydrous MeCN (1ml) is added and evaporated as before. This step is repeated again. A solution of **2f** (2mg) in anhydrous DMSO (300µl) is added. After heating at 50°C for 10 min. The crude reaction mixture is analyzed using an analytical HPLC (Column Zorbax SB C18, 50×4.6mm, 1.8µ, 2ml/min, solvent A: H₂O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product (**2g**) is confirmed by co-injection with the cold F-19 fluoro standard on the Econsphere analytical HPLC.

### Example 3

### a) 3-Fluoro-4-dimethylamino-benzoic acid methyl ester (3a)

To a stirred solution of 38,7g (225 mmol) 3.4-difluoro-benzoic acid methyl ester (Apollo) and 600 ml dimethylsulphoxid are added 22.3 g (270 mmol) dimethylamine hydrochloride and 65.4 g (473 mmol) potassium carbonate. The reaction mixture is stirred for 5h at 55°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude product **3a** is obtained in 71% yield (31.5 g, 160.0 mmol) and is used for the next step without purification.
MS-ESI: 198 (M⁺ +1, 72).

| Elementary analysis: | | | | |
|---|---|---|---|---|
| Calculated: | C 60.91% | H 6.13% | F 9.63% | N 7.10% |
| Determined: | C 60.99% | H 6.15% | F 9.60% | N 7.07% |

### b) Synthesis of (2-fluoro-4-methoxycarbonyl-phenyl)-trimethyl-ammonium trifluoromethanesulfonate (3b)

To a stirred solution of 3,90 g (19,8 mmol) **3a** and 70 ml dichloromethane are added 32.5 g (198 mmol) methyltriflate (Aldrich) drop wisely. The reaction mixture is stirred for 2.5 days at roomtemperature and diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **3b** is obtained in 80% yield (5,72 g, 15.84 mmol).
MS-ESI: 212 (M⁺, 76),

| Elementary analysis: | | | | |
|---|---|---|---|---|
| Calculated: | C 39.89% | H 4.18% | F 21.03% | N 3.88% |
| Determined: | C 39.93% | H 4.20% | F 21.01% | N 3.84% |

### c) Synthesis of (4-carboxy-2-fluoro-phenyl)-trimethyl-ammonium trifluoromethanesulfonate (3c).

A solution of 4,00 g (11.1 mmol) **3b,** 96 ml dest. water and 96 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **3c** is obtained in 92% yield (3.54 g, 10.2 mmol) and crude compound **3c** is used for the next step without purification.
MS-ESI: 198 (M⁺, 76),

| Elementary analysis: | | | | |
|---|---|---|---|---|
| Calculated: | C 38.04% | H 3.77% | F 21.88% | N 4.03% |
| Determined: | C 38.10% | H 3.79% | F 21.81% | N 4,00% |

### d) Synthesis of (4-Trimethylammonium-3-fluoro-benzoyl)-Gly-Thr-Tyr-Ala-NH₂- triflate salt (3d).

To a stirred solution of 69,4 mg (0.2 mmol) **3c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Gly-Thr(OtBu)-Tyr(O-tBu)-Ala-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 69,4 mg (0.2 mmol) **3c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**3d**) in 41.6 % yield - 20.0 mg (0.027 mmol).
MS-ESI: 590 (M⁺, 100)

### e) Synthesis of

(3-Fluoro-4-[¹⁸F]-fluoro-benzoyl)-Gly-Thr-Tyr-Ala-NH₂ (3e).

To a Wheaton vial (5ml) charged with Kryptofix 222 (5mg), potassium carbonate (1mg in 500µl water) and MeCN (1.5ml) the fluorine containing water (405 MBq, 35µl) are added. The solvent is removed by heating at 120°C for 10 mins under a stream of nitrogen. Anhydrous MeCN (1ml) is added and evaporated as before. This step is repeated again. A solution of **3d** (2mg) in anhydrous DMSO (300µl) is added. After heating at 80°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **3e** is confirmed by co-injection with the cold F-19 fluoro standard on the Econsphere analytical HPLC.

### Example 4

### a) 2-Fluoro-4-dimethylamino-benzoic acid methyl ester (4a)

To a stirred solution of 3,87 g (22.5 mmol) 2.4-difluoro-benzoic acid methyl ester (Apollo) and 60.0 ml dimethylsulphoxid are added 2.23 g (27.0 mmol) dimethylamine hydrochloride and 6.54 g (47.3 mmol) potassium carbonate. The reaction mixture is stirred for 5h at 55°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **4a** is obtained in 55% yield (2.44 g, 12.4 mmol).
MS-ESI: 198 (M⁺ +1,86).

| Elementary analysis: | | | | |
|---|---|---|---|---|
| Calculated: | C 60.91% | H 6.13% | F 9.63% | N 7.10% |
| Determined: | C 60.95% | H 6.14% | F 9.59% | N 7.08% |

### b) Synthesis of (3-fluoro-4-methoxycarbonyl-phenyl)-trimethyl-ammonium trifluoromethanesulfonate (4b)

To a stirred solution of 2.46 g (12.5 mmol) **4a** and 50 ml dichloromethane are added 20.5 g (125 mmol) methyltriflate (Aldrich) drop wisely. The reaction mixture is refluxed for 2 days. The solvent is carefully substituted by dichloroethan. The reaction mixture is refluxed for 2 days and then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **4b** is obtained in 80% yield (3.61 g, 10.0 mmol).
MS-ESI: 212 (M⁺, 77),

| Elementary analysis: | | | | |
|---|---|---|---|---|
| Calculated: | C 39.89% | H 4.18% | F 21.03% | N 3.88% |
| Determined: | C 39.94% | H 4.21% | F 21.00% | N 3.85% |

### c) Synthesis of (4-carboxy-3-fluoro-phenyl)-trimethyl-ammonium trifluoromethanesulfonate (4c).

A solution of 2.50 g (6,92 mmol) **4b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **4c** is obtained in 100% yield (2.40 g; 6,92 mmol) and crude compound **4c** is used for the next step without purification.
MS-ESI: 198 (M⁺ , 76),

| Elementary analysis: | | | | |
|---|---|---|---|---|
| Calculated: | C 38.04% | H 3.77% | F 21.88% | N 4.03% |
| Determined: | C 38.09% | H 3,80% | F 21.82% | N 4,01% |

### d) Synthesis of (4-Trimethylammonium-2-fluoro-benzoyl)-Lys(N-dimethyl)-Ala-Gly-Leu-NH₂- triflate salt (4d).

To a stirred solution of 69,4 mg (0.2 mmol) **4c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Lys(N-dimethyl)-Ala-Gly-Leu-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 69,4 mg (0.2 mmol) **4c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**4d**) in 45,0 % yield - 24,7 mg (0.0293 mmol).
MS-ESI: 596 (M⁺, 100)

### e) Synthesis of

(2-Fluoro-4-[¹⁸F]-fluoro-benzoyl)-Lys(N-dimethyl)-Ala-Gly-Leu-NH₂ (4e).

To a Wheaton vial (5ml) charged with Kryptofix 222 (5mg), potassium carbonate (1 mg in 500µl water) and MeCN (1.5ml) the fluorine containing water (385 MBq, 39µl) are added. The solvent is removed by heating at 120°C for 10 mins under a stream of nitrogen. Anhydrous MeCN (1ml) is added and evaporated as before. This step is repeated again. A solution of **4d** (2mg) in anhydrous DMSO (300µl) is added. After heating at 90°C for 10 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **4e** is confirmed by co-injection with the cold F-19 fluoro standard on the Econsphere analytical HPLC.

### Example 5

### a) 4-Dimethylamino-2.3-difluoro-benzoic acid methyl ester (5a)

To a stirred solution of 4,28 g (22.5 mmol) 2.3.4-Trifluoro-benzoic acid methyl ester (Apollo) and 60.0 ml dimethylsulphoxid are added 2.23 g (27.0 mmol) dimethylamine hydrochloride and 6.54 g (47.3 mmol) potassium carbonate. The reaction mixture is stirred for 5h at 55°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **5a** is obtained in 69 % yield (3.34 g, 15,5 mmol).
MS-ESI: 216 (M⁺ +1, 81).

| | | | | |
|---|---|---|---|---|
| Elementary analysis: | C 55.81% | H 5.15% | F 17.66% | N 6.51% |
| Determined: | C 55.90% | H 5.19% | F 17.63% | N 6,48% |

### b)Trifluoro-methanesulfonate(2,3-difluoro-4-methoxycarbonyl-phenyl)-trimethylammonium (5b)

To a stirred solution of 2.69 g (12.5 mmol) **5a** and 50 ml dichloromethane are added 20.5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **5b** is obtained in 82% yield (3,88 g, 10.3 mmol).
MS-ESI: 230 (M⁺, 34),

| Elementary analysis: | | | | |
|---|---|---|---|---|
| Calculated: | C 38.00% | H 3.72% | F 25.04% | N 3.69% |
| Determined: | C 38.04% | H 3.74% | F 25.00% | N 3.67% |

### c) Trifluoro-methanesulfonate(4-carboxy-2.3-difluoro-phenyl)-trimethyl-ammonium; (5c).

A solution of 2.63 g (6,92 mmol) **5b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **5c** is obtained in 89% yield (2.24 g; 6.16 mmol) and crude compound **5c** is used for the next step without purification.
MS-ESI: 216 (M⁺, 77),

| Elementary analysis: | | | | |
|---|---|---|---|---|
| Calculated: | C 36.17% | H 3.31% | F 26.01% | N 3.83% |
| Determined: | C 36.21% | H 3.32% | F 26.00% | N 3,81% |

### d) Synthesis of (4-Trimethylammonium-2.3-difluoro-benzoyl)-Val-Arg-Ser-Gly-NH₂-triflate salt (5d).

To a stirred solution of 73 mg (0.2 mmol) **5c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-Arg(Pbf)-Ser(OtBu)-Gly-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 73 mg (0.2 mmol) **5c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**5d**) in 39,5 % yield - 19,6 mg (0.0256 mmol).
MS-ESI: 616 (M⁺, 100)

### e) Synthesis of

(2.3-Difluoro-4-[¹⁸F]-fluoro-benzoyl)-Val-Arg-Ser-Gly-NH₂ (5e).

To a Wheaton vial (5ml) charged with Kryptofix 222 (5mg), potassium carbonate (1mg in 500µl water) and MeCN (1.5ml) the fluorine containing water (319 MBq, 35µl) are added. The solvent is removed by heating at 120°C for 10 mins under a stream of nitrogen. Anhydrous MeCN (1ml) is added and evaporated as before. This step is repeated again. A solution of **5d** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **5e** is confirmed by co-injection with the cold F-19 fluoro standard on the Econsphere analytical HPLC.

### Example 6

### a) 4-Dimethylamino-2.6-difluoro-benzoic acid methyl ester (6a)

To a stirred solution of 4,28 g (22.5 mmol) 2.4,6-Trifluoro-benzoic acid methyl ester (Apollo) and 60.0 ml dimethylsulphoxid are added 2.23 g (27.0 mmol) dimethylamine hydrochloride and 6.54 g (47.3 mmol) potassium carbonate. The reaction mixture is stirred for 5h at 55°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **6a** is obtained in 74 % yield (3.59 g, 16.7 mmol).
MS-ESI: 216 (M⁺ +1,69).

| | | | | |
|---|---|---|---|---|
| Elementary analysis: | C 55.81% | H 5.15% | F 17.66% | N 6.51% |
| Determined: | C 55.89% | H 5.18% | F 17.64% | N 6,49% |

### b)Trifluoro-methanesulfonate(2.6-difluoro-4-methoxycarbonyl-phenyl)-trimethyl-ammonium (6b)

To a stirred solution of 2.69 g (12.5 mmol) **6a** and 50 ml dichloromethane are added 20.5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **6b** is obtained in 78% yield (3.70 g, 9,75 mmol).
MS-ESI: 230 (M⁺, 55),

| Elementary analysis: | | | | |
|---|---|---|---|---|
| Calculated: | C 38.00% | H 3.72% | F 25.04% | N 3.69% |
| Determined: | C 38.05% | H 3.73% | F 25.01% | N 3.68% |

### c)Trifluoro-methanesulfonate(4-carboxy-2.6-difluoro-phenyl)-trimethyl-ammonium; (6c).

A solution of 2.63 g (6,92 mmol) **6b,** 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **6c** is obtained in 92% yield (2.38 g; 6.37 mmol) and crude compound **6c** is used for the next step without purification.
MS-ESI: 216 (M⁺ , 70),

| Elementary analysis: | | | | |
|---|---|---|---|---|
| Calculated: | C 36.17% | H 3.31% | F 26.01% | N 3.83% |
| Determined: | C 36.20% | H 3.33% | F 25.99% | N 3,82% |

### d) Synthesis of (4-Trimethylammonium-2.6-difluoro-benzoyl)-Gly-Pro-Phe-Val-NH₂-triflate salt (6d).

To a stirred solution of 73 mg (0.2 mmol) **6c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Gly-Pro-Phe-Val-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 73 mg (0.2 mmol) **6c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**6d**) in 19,6 % yield - 9,75 mg (0.0127 mmol).
MS-ESI: 616 (M⁺, 100)

### e) Synthesis of (2.6-Difluoro-4-[¹⁸F]-fluoro-benzoyl)-Gly-Pro-Phe-Val-NH₂(6e).

To a Wheaton vial (5ml) charged with Kryptofix 222 (5mg), potassium carbonate (1mg in 500µl water) and MeCN (1.5ml) the fluorine containing water (320 MBq, 37µl) are added. The solvent is removed by heating at 120°C for 10 mins under a stream of nitrogen. Anhydrous MeCN (1ml) is added and evaporated as before. This step is repeated again. A solution of **6d** (2mg) in anhydrous DMSO (300µl) is added. After heating at 90°C for 15 min. The crude reaction mixture is analyzed using an analytical HPLC (Column Zorbax SB C18, 50x4.6mm, 1.8µ, 2ml/min, solvent A: H2O, solvent B: MeCN, gradient: 5%-95% B in 7 mins or Column Econosphere C18 , 53x7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **6e** is confirmed by co-injection with the cold F-19 fluoro standard on the Econsphere analytical HPLC.

### Example 7

### a) 2-Bromo-4-dimethylamino-benzoic acid methyl ester (7a)

To a stirred solution of 5,24 g (22.5 mmol) 2-Bromo-4-fluoro-benzoic acid methyl ester (Rarechemicals) and 60.0 ml dimethylsulphoxid are added 2.23 g (27.0 mmol) dimethylamine hydrochloride and 6.54 g (47.3 mmol) potassium carbonate. The reaction mixture is stirred for 11h at 70°C in an autoclave and is concentrated with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **7a** is obtained in 70 % yield (4,08 g, 15.8 mmol).
MS-ESI: 258/560 (M⁺ +1, 88/83).

| | | | |
|---|---|---|---|
| Elementary analysis: | C 46.53% | H 4.69% | N 5.43% |
| Determined: | C 46,60% | H 4.71% | N 5.42% |

### b) Trifluoro-methanesulfonate(3-bromo-4-methoxycarbonyl-phenyl)-trimethyl-ammonium (7b)

To a stirred solution of 2,69 g (12,5 mmol) **7a** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **7b** is obtained in 69% yield (3,66 g, 8,63 mmol).
MS-ESI: 273/275 (M⁺ +1, 78/72),

| Elementary analysis: | | | |
|---|---|---|---|
| Calculated: | C 34.14% | H 3.58% F 13.50% | N 3.32% |
| Determined: | C 34.17% | H 3.59% F 13.47% | N 3.31% |

### c) Trifluoro-methanesulfonate(3-bromo-4-carboxy-phenyl)-trimethyl-ammonium; (7c).

A solution of 2,92 g (6,92 mmol) **7b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **7c** is obtained in 87% yield (2,46 g; 6.02 mmol) and crude compound 7c is used for the next step without purification.
MS-ESI: 258/260 (M⁺, 64/59),

| Elementary analysis: | | | | |
|---|---|---|---|---|
| Calculated: | C 32.37% | H 3.21% | F 13.96% | N 3.43% |
| Determined: | C 32.41% | H 3,22% | F 13.94% | N 3,42% |

### d) Synthesis of (4-Trimethylammonium-2-bromo-benzoyl)-Gly-Phe-lle-Gly-NH₂-triflate salt (7d).

To a stirred solution of 81.6 mg (0.2 mmol) **7c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Gly-Phe-Ile-Gly-NH2 (loading 0.68 mmol/g) which is prepared by standard protocol. The mixture is shaken intensively for 4h. The mixture is filtered and washed with dimethylformamide.. The coupling step is repeated. Thus, to a stirred solution of 81.6 mg (0.2 mmol) **7c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**7d**) in 24,6 % yield - 12,5 mg (0,016 mmol).
MS-ESI: 633/635 (M⁺, 100/88)

### e) Synthesis of [¹⁸F]-(2-Bromo-4-fluoro-benzoyl)-Gly-Phe-Ile-Gly-NH₂ (7e).

To a Wheaton vial (5ml) charged with Kryptofix 222 (5mg), potassium carbonate (1mg in 500µl water) and MeCN (1.5ml) the fluorine containing water (336 MBq, 35µl) are added. The solvent is removed by heating at 120°C for 10 mins under a stream of nitrogen. Anhydrous MeCN (1ml) is added and evaporated as before. This step is repeated again. A solution of **7d** (2mg) in anhydrous DMSO (300µl) is added. After heating at 90°C for 12 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **7e** is confirmed by co-injection with the cold F-19 fluoro standard on the Econsphere analytical HPLC.

### Example 8

### a) 4-Dimethylamino-2-nitro-benzoic acid methyl ester (8a)

To a stirred solution of 4,48 g (22.5 mmol) 4-fluoro-2-nitro-benzoic acid methyl ester (J. Fluorine Chem.; 63; 1-2; (1993); 25-30) and 60.0 ml dimethylsulphoxid are added 2.23 g (27.0 mmol) dimethylamine hydrochloride and 6.54 g (47.3 mmol) potassium carbonate. The reaction mixture is stirred for 7h at 60°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **8a** is obtained in 61 % yield (3,08 g, 13,7 mmol).
MS-ESI: 225 (M⁺ +1, 71).

| | | | |
|---|---|---|---|
| Elementary analysis: | C 53.57% | H 5.39% | N 12.49% |
| Determined: | C 53.60% | H 5.40% | N 12,47% |

### b) Trifluoro-methanesulfonate(4-methoxycarbonyl-3-nitro-phenyl)-trimethyl -ammonium (8b)

To a stirred solution of 2,80 g (12,5 mmol) **8a** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **8b** is obtained in 45% yield (2,18 g, 5,63 mmol).
MS-ESI: 239 (M⁺ , 89),

| Elementary analysis: | | | | |
|---|---|---|---|---|
| Calculated: | C 37.12% | H 3.89% | F 14.68% | N 7.21% |
| Determined: | C 37.15% | H 3.90% | F 14.67% | N 7.19% |

### c) Trifluoro-methanesulfonate(4-carboxy-3-nitro-phenyl)-trimethyl-ammonium (8c).

A solution of 2.68 g (6,92 mmol) **8b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 96% yield (2,48 g; 6.64 mmol) and crude compound **8c** is used for the next step without purification.
MS-ESI: 225 (M⁺, 66),

| Elementary analysis: | | | | |
|---|---|---|---|---|
| Calculated: | C 35.30% | H 3.50% | F 15.23% | N 7.48% |
| Determined: | C 35.31% | H 3,50% | F 15.23% | N 7,47% |

### d) Synthesis of (4-Trimethylammonium-2-nitro-benzoyl)-Ser-Thr-Val-Gly-NH₂-triflate salt (8d).

To a stirred solution of 75,0 mg (0.2 mmol) **8c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Ser(Ot-Bu)-Thr(OtBu)-Val-Gly-NH2 (loading 0.68 mmol/g) which is prepared by standard protocol. The mixture is shaken intensively for 4h. The mixture is filtered and washed with dimethylformamide. The coupling step is repeated. Thus, to a stirred solution of 75,0 mg (0.2 mmol) **8c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**8d**) in 35% yield (16,3 mg, 0,0228 mmol).
MS-ESI: 569 (M⁺, 100)

### e) Synthesis of [¹⁸F]-(2-Nitro-4-fluoro-benzoyl)-Ser-Thr-Val-Gly-NH₂ (8e).

To a Wheaton vial (5ml) charged with Kryptofix 222 (5mg), potassium carbonate (1mg in 500µl water) and MeCN (1.5ml) the fluorine containing water (376 MBq, 35µl) is added. The solvent is removed by heating at 120°C for 10 mins under a stream of nitrogen. Anhydrous MeCN (1ml) is added and evaporated as before. This step is repeated again. A solution of **8d** (2mg) in anhydrous DMSO (300µl) is added. After heating at 80°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **8e** is confirmed by co-injection with the cold F-19 fluoro standard on the Econsphere analytical HPLC.

### Example 9

### a) 2-Cyano-4-dimethylamino-benzoic acid methyl ester (9a)

To a stirred solution of 4,03 g (22.5 mmol) 2-Cyano-4-fluoro-benzoic acid methyl ester (J. Med. Chem., 35; 24; (1992); 4613-4627) and 60.0 ml dimethylsulphoxid are added 2.23 g (27.0 mmol) dimethylamine hydrochloride and 6.54 g (47.3 mmol) potassium carbonate. The reaction mixture is stirred for 9h at 60°C in an autoclave and is concentrated with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **9a** is obtained in 85% yield (3,90 g, 19.1 mmol).
MS-ESI: 205 (M⁺ +1, 81).

| | | | |
|---|---|---|---|
| Elementary analysis: | C 64.69% | H 5.92% | N 13.72% |
| Determined: | C 64.72% | H 5.95% | N 13,70% |

### b) Trifluoro-methanesulfonate(3-cyano-4-methoxycarbonyl-phenyl)-trimethyl -ammonium; (9b)

To a stirred solution of 2,55 g (12,5 mmol) **9a** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **9b** is obtained in 78% yield (3,59 g, 9,75 mmol).
MS-ESI: 219 (M⁺ +1, 79),

| Elementary analysis: | | | | |
|---|---|---|---|---|
| Calculated: | C 42.39% | H 4.10% | F 15.47% | N 7.61% |
| Determined: | C 42.41% | H 4.11% | F 15.42% | N 7.60% |

### c) Trifluoro-methanesulfonate(4-carboxy-3-cyano-phenyl)-trimethyl-ammonium; (9c).

A solution of 2,55 g (6,92 mmol) **9b,** 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 86% yield (2,10 g; 5.95 mmol) and crude compound **9c** is used for the next step without purification.
MS-ESI: 205 (M⁺, 76),

| Elementary analysis: | | | | |
|---|---|---|---|---|
| Calculated: | C 40.68% | H 3.70% | F 16.09% | N 7.91% |
| Determined: | C 40.69% | H 3,71% | F 16.07% | N 7,90% |

### d) Synthesis of (4-Trimethylammonium-2-cyano-benzoyl)-Arg-Val-Gly-Phe-NH₂- triflate salt (9d).

To a stirred solution of 71.0 mg (0.2 mmol) **9c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Arg(Pbf)-Val-Gly-Phe-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 71.0 mg (0.2 mmol) **9c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**9d**) obtained in 38% yield (20.0 mg, 0.0247 mmol).
MS-ESI: 665 (M⁺, 100)

### e) Synthesis of [¹⁸F]-(2-Cyano-4-fluoro-benzoyl)-Arg-Val-Gly-Phe-NH₂ (9e).

To a Wheaton vial (5ml) charged with Kryptofix 222 (5mg), potassium carbonate (1mg in 500µl water) and MeCN (1.5ml) the fluorine containing water (309 MBq, 35µl) is added. The solvent is removed by heating at 120°C for 10 mins under a stream of nitrogen. Anhydrous MeCN (1ml) is added and evaporated as before. This step is repeated again. A solution of **9d** (2mg) in anhydrous DMSO (300µl) is added. After heating at 80°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **9e** is confirmed by co-injection with the non-radioactive F-19 fluoro standard on the Econsphere analytical HPLC.

### Example 10

### a) 4-Dimethylamino-3-methanesulfonyl-benzoic acid methyl ester (10a)

To a stirred solution of 5,23 g (22.5 mmol) 4-Fluoro-3-methanesulfonyl-benzoic acid methyl ester (J. Med. Chem.; 40; 13; 1997; 2017-2034) and 60.0 ml dimethylsulphoxid are added 2.23 g (27.0 mmol) dimethylamine hydrochloride and 6.54 g (47.3 mmol) potassium carbonate. The reaction mixture is stirred for 7h at 60°C in an autoclave and is concentrated with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **10a** is obtained in 56 % yield (3,24 g, 12,6 mmol).
MS-ESI: 258 (M⁺ +1, 81).

| | | | |
|---|---|---|---|
| Elementary analysis: | C 51.35% | H 5.88% | N 5.44% |
| Determined: | C 51.37% | H 5.90% | N 5.42% |

### b) Trifluoro-methanesulfonate(2-methanesulfonyl-4-methoxycarbonyl-phenyl) -trimethyl-ammonium, (10b)

To a stirred solution of 3,22 g (12,5 mmol) **10a** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **10b** is obtained in 58% yield (3,05 g, 7,25 mmol).
MS-ESI: 272 (M⁺, 88),

| Elementary analysis: | | | | |
|---|---|---|---|---|
| Calculated: | C 37.05% | H 4.31% | F 13.52% | N 3.32% |
| Determined: | C 37.09% | H 4.33% | F 13.50% | N 3.31% |

### c) Trifluoro-methanesulfonate(4-carboxy-2-methanesulfonyl-phenyl)-trimethylammonium; (10c).

A solution of 2,91 g (6,92 mmol) **10b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 96% yield (2,70 g; 6.64 mmol) and crude compound **10c** is used for the next step without purification.
MS-ESI: 258 (M⁺ , 93),

| Elementary analysis: | | | | |
|---|---|---|---|---|
| Calculated: | C 35.38% | H 3.96% | F 13.99% | N 3.44% |
| Determined: | C 35.39% | H 3,96% | F 13.97% | N 3,44% |

### d) Synthesis of (4-Trimethylammonium-3-methanesulfonyl-benzoyl)-Gly-Phe-Val-Leu-NH₂- triflate salt (10d).

To a stirred solution of 81.0 mg (0.2 mmol) **10c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Gly-Phe-Val-Leu-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 81.0 mg (0.2 mmol) **10c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water : acetonitril gradient and 0.1% trifluoro acetic acid as co-solvent to obtain the desired product **(10d)** in 33 % yield - 17,7 mg (0.021 mmol).
MS-ESI: 675 (M⁺, 100)

### e) Synthesis of [¹⁸F]-(3-Methanesulfonyl-4-fluoro-benzoyl)-Gly-Phe-Val-Leu-NH₂ (10e).

To a Wheaton vial (5ml) charged with Kryptofix 222 (5mg), potassium carbonate (1mg in 500µl water) and MeCN (1.5ml) the fluorine containing water (399 MBq, 35µl) is added. The solvent is removed by heating at 120°C for 10 mins under a stream of nitrogen. Anhydrous MeCN (1ml) is added and evaporated as before. This step is repeated again. A solution of **10d** (2mg) in anhydrous DMSO (300µl) is added. After heating at 80°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **10e** is confirmed by co-injection with the non-radioactive F-19 fluoro standard on the Econsphere analytical HPLC.

### Example 11

### a) 4-Dimethylamino-3-nitro-benzoic acid methyl ester (11a)

To a stirred solution of 4,48 g (22.5 mmol) 4-Fluoro-3-nitro-benzoic acid methyl ester (Bioorg. Med. Chem.; 6; 8; 1998; 1185 - 1208) and 60.0 ml dimethylsulphoxid are added 2.23 g (27.0 mmol) dimethylamine hydrochloride and 6.54 g (47.3 mmol) potassium carbonate. The reaction mixture is stirred for 8h at 60°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **11a** is obtained in 69 % yield (3,48 g, 15,5 mmol).
MS-ESI: 225 (M⁺ +1, 74).

| | | | |
|---|---|---|---|
| Elementary analysis: | C 53.57% | H 5.39% | N 12.49% |
| Determined: | C 53.62% | H 5.42% | N 12,46% |

### b) Trifluoro-methanesulfonate(4-methoxycarbonyl-2-nitro-phenyl)-trimethyl -ammonium (11b)

To a stirred solution of 2,80 g (12,5 mmol) **11a** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **11b** is obtained in 71% yield (3,44 g, 8,88 mmol).
MS-ESI: 239 (M⁺, 82),

| Elementary analysis: | | | | |
|---|---|---|---|---|
| Calculated: | C 37.12% | H 3.89% | F 14.68% | N 7.21% |
| Determined: | C 37.14% | H 3.91% | F 14.67% | N 7.20% |

### c) Trifluoro-methanesulfonate(4-carboxy-2-nitro-phenyl)-trimethyl-ammonium (11c).

A solution of 2.68 g (6,92 mmol) **11b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 93% yield (2,41 g; 6.44 mmol) and crude compound **11c** is used for the next step without purification.
MS-ESI: 225 (M⁺ , 66),

| Elementary analysis: | | | | |
|---|---|---|---|---|
| Calculated: | C 35.30% | H 3.50% | F 15.23% | N 7.48% |
| Determined: | C 35.32% | H 3,51% | F 15.21% | N 7,46% |

### d) Synthesis of (4-Trimethylammonium-3-nitro-benzoyl)-Thr-Val-Phe-Leu-NH₂- triflate salt (11d).

To a stirred solution of 75,0 mg (0.2 mmol) **11c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Thr(OtBu)-Val-Phe-Leu-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 75,0 mg (0.2 mmol) **11c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **(11d)** in 56% yield (30.1mg, 0.0364 mmol).
MS-ESI: 686 (M⁺, 100)

### e) Synthesis of [¹⁸F]-(3-Nitro-4-fluoro-benzoyl)- Thr-Val-Phe-Leu-NH₂ (11e).

To a Wheaton vial (5ml) charged with Kryptofix 222 (5mg), potassium carbonate (1mg in 500µl water) and MeCN (1.5ml) the fluorine containing water (344 MBq, 35µl) is added. The solvent is removed by heating at 120°C for 10 mins under a stream of nitrogen. Anhydrous MeCN (1ml) is added and evaporated as before. This step is repeated again. A solution of **11d** (2mg) in anhydrous DMSO (300µl) is added. After heating at 65°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **11e** is confirmed by co-injection with the non-radioactive F-19 fluoro standard on the Econsphere analytical HPLC.

### Example 12

### a) 4-Dimethylamino-3-trifluoromethyl-benzoic acid methyl ester (12a)

To a stirred solution of 4,48 g (22.5 mmol) 4-Fluoro-3-trifluoromethyl-benzoic acid methyl ester (Rarechem) and 60.0 ml dimethylsulphoxid are added 2.23 g (27.0 mmol) dimethylamine hydrochloride and 6.54 g (47.3 mmol) potassium carbonate. The reaction mixture is stirred for 8h at 60°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **12a** is obtained in 72 % yield (4,00 g, 16,2 mmol).
MS-ESI: 248 (M⁺ +1, 100).

| | | | | |
|---|---|---|---|---|
| Elementary analysis: | C 53.44% | H 4.89% | F 23.05% | N 5.67% |
| Determined: | C 53.48% | H 4.90% | F 23.03% | N 5.65% |

### b) Trifluoro-methanesulfonate(4-methoxycarbonyl-2-trifluoromethyl-phenyl)-trimethylammonium (12b)

To a stirred solution of 3.09 g (12.5 mmol) **12a** and 50 ml dichloromethane are added 20.5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **12b** is obtained in 69% yield (3.55 g, 8,63 mmol).
MS-ESI: 262 (M⁺, 67),

| Elementary analysis: | | | | |
|---|---|---|---|---|
| Calculated: | C 37.96% | H 3.68% | F 27.71% | N 3.41% |
| Determined: | C 38.00% | H 3.62% | F 27.68% | N 3.40% |

### c) Trifluoro-methanesulfonate(4-carboxy-2-trifluoromethyl-phenyl)-trimethyl-ammonium (12c).

A solution of 2.84 g (6,92 mmol) **12b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 89% yield (2.45 g; 6.16 mmol) and crude compound **12c** is used for the next step without purification.
MS-ESI: 248 (M⁺, 100),

| Elementary analysis: | | | | |
|---|---|---|---|---|
| Calculated: | C 36.28% | H 3.30% | F 28.69% | N 3.53% |
| Determined: | C 36.29% | H 3.31% | F 28.67% | N 3.51% |

### d) Synthesis of (4-Trimethylammonium-3-trifluormethyl-benzoyl)-Val-ßAla-Phe-Gly-NH₂- triflate salt (12d).

To a stirred solution of 79,4 mg (0.2 mmol) **12c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Phe-Gly-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 79,4 mg (0.2 mmol) **12c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**12d**) in 21.5 % yield - 10.9 mg (0.014 mmol).
MS-ESI: 636 (M⁺, 100)

### e) Synthesis of [¹⁸F]-(3-Trifluormethyl-4-fluoro-benzoyl)-Val-βAla-Phe-Gly-NH₂ (12e).

To a Wheaton vial (5ml) charged with Kryptofix 222 (5mg), potassium carbonate (1mg in 500µl water) and MeCN (1.5ml) the fluorine containing water (356 MBq, 35µl) is added. The solvent is removed by heating at 120°C for 10 mins under a stream of nitrogen. Anhydrous MeCN (1ml) is added and evaporated as before. The step is repeated again. A solution of **12d** (2mg) in anhydrous DMSO (300µl) is added. After heating at 50°C for 18 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H₂O + 0.1%TFA, solvent B: MeCN/Water 9/1 + 0.1% TFA, gradient: 5-95% B in 7 mins. The F-18 labeled product **12e** is confirmed by co-injection with the non-radioactive F-19 fluoro standard on the Econsphere analytical HPLC.

### Example 13

### a) 4-Dimethylamino-2-trifluoromethyl-benzoic acid methyl ester (13a)

To a stirred solution of 4,48 g (22.5 mmol) 4-Fluoro-2-trifluoromethyl-benzoic acid methyl ester (Rarechem) and 60.0 ml dimethylsulphoxid are added 2.23 g (27.0 mmol) dimethylamine hydrochloride and 6.54 g (47.3 mmol) potassium carbonate. The reaction mixture is stirred for 8h at 60°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **13a** is obtained in 72 % yield (4,00 g, 16,2 mmol).
MS-ESI: 248 (M⁺ +1,78).

| | | | | |
|---|---|---|---|---|
| Elementary analysis: | C 53.44% | H 4.89% | F 23.05% | N 5.67% |
| Determined: | C 53.46% | H 4.91% | F 23,04% | N 5.64% |

### b) Trifluoro-methanesulfonate(4-methoxycarbonyl-3-trifluoromethyl-phenyl)-trimethylammonium (13b)

To a stirred solution of 3,09 g (12,5 mmol) **13a** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **13b** is obtained in 69% yield (3,55 g, 8,63 mmol).
MS-ESI: 262 (M⁺ , 87),

| Elementary analysis: | | | | |
|---|---|---|---|---|
| Calculated: | C 37.96% | H 3.68% | F 27.71% | N 3.41% |
| Determined: | C 38.01% | H 3.63% | F 27.69% | N 3.41% |

### c) Trifluoro-methanesulfonate(4-carboxy-3-trifluoromethyl-phenyl)-trimethylammonium (13c).

A solution of 2,84 g (6,92 mmol) **13b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 89% yield (2,45 g; 6.16 mmol) and crude compound **13c** is used for the next step without purification.
MS-ESI: 248 (M⁺, 59),

| Elementary analysis: | | | | |
|---|---|---|---|---|
| Calculated: | C 36.28% | H 3.30% | F 28.69% | N 3.53% |
| Determined: | C 36.30% | H 3,32% | F 28.67% | N 3,52% |

### d) Synthesis of (4-Trimethylammonium-2-trifluormethyl-benzoyl)-Val-ßAla-His(π-Me)-Gly-NH₂-triflate salt (13d)

To a stirred solution of 79,4 mg (0.2 mmol) **13c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-His(π-Me)-Gly-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 79,4 mg (0.2 mmol) **13c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**13d**).
MS-ESI: 640 (M⁺, 100)

### e) Synthesis of ([¹⁸F]-4-Fluoro-2-trifluormethyl-benzoyl)-Val-βAla-His(π-Me)-Gly-NH₂ (13e)

To a Wheaton vial (5ml) charged with Kryptofix 222 (5mg), potassium carbonate (1mg in 500µl water) and MeCN (1.5ml) the fluorine containing water (321 MBq, 35µl) is added. The solvent is removed by heating at 120°C for 10 mins under a stream of nitrogen. Anhydrous MeCN (1ml) is added and evaporated as before. This step is repeated again. A solution of **13d** (2mg) in anhydrous DMSO (300µl) is added. After heating at 75°C for 20 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **13e** is confirmed by co-injection with the non-radioactive F-19 fluoro standard on the Econsphere analytical HPLC.

### Example 14

### a) 4-Fluoro-3-trifluoromethoxy-benzoic acid methyl ester (14a).

To a stirred suspension of 21,2g (96,9 mmol) 4-Fluoro-3-trifluoromethoxy-benzoic acid (JRD-Fluoro) and 161 ml methanol are added 30.4 g (387,6 mmol) acetyl chloride drop wisely at 0°C. The reaction mixture is stirred over night, filtered and concentrated. The residue is diluted with dichloromethane, washed with diluted sodium hydrogen carbonate solution, dried with sodium sulphate and concentrated. The residue is purified by column chromatography (hexane : ethylacetate). The desired product **14a** is obtained in 75% yield (17.3 g; 72.7 mmol)
MS-ESI: 239 (M⁺ +1, 66),

| Elementary analysis: | | | |
|---|---|---|---|
| Calculated: | C 45.39% | H 2.54% | F 31.91% |
| Determined: | C 45.41% | H 2,52% | F 31.89% |

### b) 4-Dimethylamino-3-trifluoromethoxy-benzoic acid methyl ester (14b)

To a stirred solution of 5.36 g (22.5 mmol) **14a** and 60.0 ml dimethylsulphoxid are added 2.23 g (27.0 mmol) dimethylamine hydrochloride and 6.54 g (47.3 mmol) potassium carbonate. The reaction mixture is stirred for 8h at 60°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **14b** is obtained in 69 % yield (4,09 g, 15.5 mmol).
MS-ESI: 264 (M⁺ +1,100).

| | | | | |
|---|---|---|---|---|
| Elementary analysis: | C 50.20% | H 4.60% | F 21.65% | N 5.32% |
| Determined: | C 50.22% | H 4.61% | F 21,64% | N 5.31% |

### c) Trifluoro-methanesulfonate(4-methoxycarbonyl-2-trifluoromethoxy-phenyl)-trimethylammonium (14c)

To a stirred solution of 3,29 g (12,5 mmol) **14b** and 50 ml dichloroethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **14c** is obtained in 57% yield (3,06 g, 7,13 mmol).
MS-ESI: 278 (M⁺, 82),

| Elementary analysis: | | | | |
|---|---|---|---|---|
| Calculated: | C 36.54% | H 3.54% | F 26.68% | N 3.28% |
| Determined: | C 36.56% | H 3.56% | F 27.67% | N 3.26% |

### d) Trifluoro-methanesulfonate(4-carboxy-2-trifluoromethoxy-phenyl)-trimethyl-ammonium (14d).

A solution of 2,95g (6,92 mmol) **14c**, 60 ml dest. water and 60 ml trifluoroacetic acid was refluxed for 2 days. The reaction mixture was evaporated, dried by use of oil pump vacuum over night and treated with diethyl ether. The resulting solid was filtered, washed extensively with diethyl ether and dried by oil pump vacuum. The solid crude was obtained in 94% yield (2,68 g; 6.50 mmol) and crude compound **14d** was used for the next step without purification.
MS-ESI: 264 (M⁺, 100),

| Elementary analysis: | | | | |
|---|---|---|---|---|
| Calculated: | C 34.87% | H 3.17% | F 27.58% | N 3.39% |
| Determined: | C 34.89% | H 3,19% | F 27.56% | N 3,38% |

### e) Synthesis of (4-Trimethylammonium-3-trifluormethoxy-benzoyl)-Val-βAla-Phe-Gly-NH₂- triflate salt (14e)

To a stirred solution of 82.6 mg (0.2 mmol) **14d** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-βAla-Phe-Gly-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 82.6 mg (0.2 mmol) **14d** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**14e**) in 28 % yield - 14,6 mg (0.0182 mmol).
MS-ESI: 653 (M⁺, 100)

### f) Synthesis of ([¹⁸F]-4-Fluoro-3-trifluormethoxy-benzoyl)-Val-ßAla-Phe-Gly-NH₂ (14f)

To a Wheaton vial (5ml) charged with Kryptofix 222 (5mg), potassium carbonate (1mg in 500µl water) and MeCN (1.5ml) the fluorine containing water (321 MBq, 35µl) is added. The solvent is removed by heating at 120°C for 10 mins under a stream of nitrogen. Anhydrous MeCN (1ml) is added and evaporated as before. This step is repeated again. A solution of **14e** (2mg) in anhydrous DMSO (300µl) is added. After heating at 65°C for 10 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H₂O + 0.1%TFA, solvent B: MeCN/Water 9/1 + 0.1% TFA, gradient: 5-95% B in 7 mins. The F-18 labeled product **14f** is confirmed by co-injection with the non-radioactive F-19 fluoro standard on the Econsphere analytical HPLC.

### Example 15

### a) 5-Dimethylamino-2-trifluoromethyl-benzoic acid methyl ester (15a)

To a stirred solution of 4,48 g (22.5 mmol) 5-Fluoro-2-trifluoromethyl-benzoic acid methyl ester (Rarechem) and 60.0 ml dimethylsulphoxid are added 2.23 g (27.0 mmol) dimethylamine hydrochloride and 6.54 g (47.3 mmol) potassium carbonate. The reaction mixture is stirred for 8h at 60°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **15a** is obtained in 72 % yield (4,00 g, 16,2 mmol).
MS-ESI: 248 (M⁺ +1, 100).

| | | | | |
|---|---|---|---|---|
| Elementary analysis: | C 53.44% | H 4.89% | F 23.05% | N 5.67% |
| Determined: | C 53.45% | H 4.90% | F 23,05% | N 5.65% |

### b) Trifluoro-methanesulfonate(3-methoxycarbonyl-4-trifluoromethyl-phenyl)-trimethylammonium (15b)

To a stirred solution of 3,09 g (12,5 mmol) **15a** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **15b** is obtained in 69% yield (3,55 g, 8,63 mmol).
MS-ESI: 262 (M⁺ 100),

| Elementary analysis: | | | | |
|---|---|---|---|---|
| Calculated: | C 37.96% | H 3.68% | F 27.71% | N 3.41% |
| Determined: | C 38.00% | H 3.69% | F 27.68% | N 3.40% |

### c) Trifluoro-methanesulfonate(3-carboxy-4-trifluoromethyl-phenyl)-trimethyl-ammonium (15c).

A solution of 2,84 g (6,92 mmol) **15b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 89% yield (2,45 g; 6.16 mmol) and crude compound **15c** is used for the next step without purification.
MS-ESI: 248 (M⁺, 45),

| Elementary analysis: | | | | |
|---|---|---|---|---|
| Calculated: | C 36.28% | H 3.30% | F 28.69% | N 3.53% |
| Determined: | C 36.31% | H 3,31% | F 28.68% | N 3,51% |

### d) Synthesis of (5-Trimethylammonium-2-trifluormethyl-benzoyl)-Val-ßAla-Trp-Gly-NH₂-triflate salt (15d)

To a stirred solution of 79,4 mg (0.2 mmol) **15c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Trp(N-Boc)-Gly-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 79,4 mg (0.2 mmol) **15c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water : acetonitril gradient and 0.1% trifluoro acetic acid as co-solvent to obtain the desired product **(15d)** in 26 % yield - 13,9 mg (0.017 mmol).
MS-ESI: 675 (M⁺, 100)

### e) Synthesis of ([¹⁸F]-5-Fluoro-2-trifluormethyl-benzoyl)-Val-βAla-Trp-Gly-NH₂ (15e).

To a Wheaton vial (5ml) charged with Kryptofix 222 (5mg), potassium carbonate (1mg in 500µl water) and MeCN (1.5ml) the fluorine containing water (316MBq, 33µl) is added. The solvent is removed by heating at 120°C for 10 mins under a stream of nitrogen. Anhydrous MeCN (1ml) is added and evaporated as before. This step is repeated again. A solution of **15d** (2mg) in anhydrous DMSO (300µl) is added. After heating at 75°C for 18 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **15e** is confirmed by co-injection with the non-radioactive F-19 fluoro standard on the Econsphere analytical HPLC.

### Example 16

### a) 2-Bromo-5-dimethylamino-benzoic acid methyl ester (16a)

To a stirred solution of 5,24 g (22.5 mmol) 2-Bromo-5-fluoro-benzoic acid methyl ester (Rarechemicals) and 60.0 ml dimethylsulphoxid are added 2.23 g (27.0 mmol) dimethylamine hydrochloride and 6.54 g (47.3 mmol) potassium carbonate. The reaction mixture is stirred for 11h at 70°C in an autoclave and is concentrated with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **16a** is obtained in 70 % yield (4,08 g, 15.8 mmol).
MS-ESI: 258/560 (M⁺ +1, 90/81).

| | | | |
|---|---|---|---|
| Elementary analysis: | C 46.53% | H 4.69% | N 5.43% |
| Determined: | C 46,59% | H 4.72% | N 5.41% |

### b) Trifluoro-methanesulfonate(4-bromo-3-methoxycarbonyl-phenyl)-trimethyl -ammonium (16b)

To a stirred solution of 2,69 g (12,5 mmol) **16a** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **16b** is obtained in 79% yield (4,17 g, 9,88 mmol).
MS-ESI: 272/274 (M⁺ , 89/80),

| Elementary analysis: | | | | |
|---|---|---|---|---|
| Calculated: | C 34.14% | H 3.58% | F 13.50% | N 3.32% |
| Determined: | C 34.16% | H 3.60% | F 13.48% | N 3.30% |

### c) Trifluoro-methanesulfonate(4-bromo-3-carboxy-phenyl)-trimethyl-ammonium (16c).

A solution of 2,92 g (6,92 mmol) **16b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **16c** is obtained in 87% yield (2,46 g; 6.02 mmol) and crude compound **16c** is used for the next step without purification.
MS-ESI: 258/260 (M⁺ , 78/69),

| Elementary analysis: | | | | |
|---|---|---|---|---|
| Calculated: | C 32.37% | H 3.21% | F 13.96% | N 3.43% |
| Determined: | C 32.40% | H 3,22% | F 13.95% | N 3,41% |

### d) Synthesis of (5-Trimethylammonium-2-bromo-benzoyl)-Val-ßAla-Arg-Gly-NH₂- triflate salt (16d).

To a stirred solution of 81.4 mg (0.2 mmol) **16c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Arg(Pbf)-Gly-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 81.4 mg (0.2 mmol) **16c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**16d**) in 52% yield (27,2 mg, 0,0338 mmol).
MS-ESI: 655/657 (M⁺, 100/82)

### e) Synthesis of [¹⁸F]-(2-Bromo-5-fluoro-benzoyl)-Val-βAla-Arg-Gly-NH₂ (16e).

To a Wheaton vial (5ml) charged with Kryptofix 222 (5mg), potassium carbonate (1 mg in 500µl water) and MeCN (1.5ml) the fluorine containing water (334 MBq, 35µl) is added. The solvent is removed by heating at 120°C for 10 mins under a stream of nitrogen. Anhydrous MeCN (1ml) is added and evaporated as before. This step is repeated again. A solution of **16d** (2mg) in anhydrous DMSO (300µl) is added. After heating at 90°C for 20 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **16e** is confirmed by co-injection with the non-radioactive F-19 fluoro standard on the Econsphere analytical HPLC.

### Example 17

### a) 5-Dimethylamino-2-methanesulfonyl-benzoic acid methyl ester (17a)

To a stirred solution of 5,23 g (22.5 mmol) 5-Fluoro-2-methanesulfonyl-benzoic acid methyl ester (J. Med. Chem.; 40; 13; 1997; 2017-2034) and 60.0 ml dimethylsulphoxid are added 2.23 g (27.0 mmol) dimethylamine hydrochloride and 6.54 g (47.3 mmol) potassium carbonate. The reaction mixture is stirred for 7h at 60°C in an autoclave and is concentrated with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **17a** is obtained in 56 % yield (3,24 g, 12,6 mmol).
MS-ESI: 257 (M⁺ +1, 75).

| | | | |
|---|---|---|---|
| Elementary analysis: | C 51.35% | H 5.88% | N 5.44% |
| Determined: | C 51.37% | H 5.88% | N 5,42% |

### b) Trifluoro-methanesulfonate(4-methanesulfonyl-3-methoxycarbonyl-phenyl)-trimethylammonium (17b).

To a stirred solution of 3,22 g (12,5 mmol) **17a** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **17b** is obtained in 58% yield (3,05 g, 7,25 mmol).
MS-ESI: 272 (M⁺, 69),

| Elementary analysis: | | | | |
|---|---|---|---|---|
| Calculated: | C 37.05% | H 4.31% | F 13.52% | N 3.32% |
| Determined: | C 37.07% | H 4.33% | F 13.48% | N 3.31% |

### c) Trifluoro-methanesulfonate(3-carboxy-4-methanesulfonyl-phenyl)-trimethylammonium; (17c).

A solution of 2,91 g (6,92 mmol) **17b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 96% yield (2,70 g; 6.64 mmol) and crude compound **17c** is used for the next step without purification.
MS-ESI: 258 (M⁺ +1, 69),

| Elementary analysis: | | | | |
|---|---|---|---|---|
| Calculated: | C 35.38% | H 3.96% | F 13.99% | N 3.44% |
| Determined: | C 35.40% | H 3,97% | F 13.96% | N 3,43% |

### d) Synthesis of (5-Trimethylammonium-2-methanesulfonyl-benzoyl)-Val-ßAla-Arg-Gly-NH₂- triflate salt (17d).

To a stirred solution of 81.0 mg (0.2 mmol) **17c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Arg(Pbf)-Gly-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 81.0 mg (0.2 mmol) **17c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**17d**) in 33% yield (17,2 g, 0.0214 mmol).
MS-ESI: 656 (M⁺, 100)

### e) Synthesis of [¹⁸F]-(2-Methanesulfonyl-5-fluoro-benzoyl)-Val-ßAla-Arg-Gly-NH₂ (17e).

To a Wheaton vial (5ml) charged with Kryptofix 222 (5mg), potassium carbonate (1mg in 500µl water) and MeCN (1.5ml) the fluorine containing water (366 MBq, 35µl) is added. The solvent is removed by heating at 120°C for 10 mins under a stream of nitrogen. Anhydrous MeCN (1ml) is added and evaporated as before. This step is repeated again. A solution of **17d** (2mg) in anhydrous DMSO (300µl) is added. After heating at 70°C for 18 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **17e** is confirmed by co-injection with the non-radioactive F-19 fluoro standard on the Econsphere analytical HPLC.

### Example 18

### a) 2-Chloro-5-dimethylamino-benzoic acid methyl ester (18a)

To a stirred solution of 4,00g (20,6 mmol) 2-Chloro-5-fluoro-benzoic acid methyl ester (Rarechem) and 60 ml dimethylsulphoxid are added 2,03 g (24,7 mmol) dimethylamine hydrochloride and 5,97 g (43,2 mmol) potassium carbonate. The reaction mixture is stirred over night and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude product **18a** is obtained in 99% yield (4,46 g, 20,9 mmol) and is used for the next step without purification.
MS-ESI: 213/215 (M⁺ +1, 78 / 53).

| Elementary analysis: | | | |
|---|---|---|---|
| Calculated: | C 56.21% | H 5.66% | N 6.56% |
| Determined: | C 56.29% | H 5,68% | N 6.55% |

### b) Synthesis of (4-chloro-3-methoxycarbonyl-phenyl)-trimethyl-ammonium trifluoromethanesulfonate (18b)

To a stirred solution of 4.49 g (21.0 mmol) **18a** and 75 ml dichloromethane are added 34,5 g (21.0 mmol) methyltriflate (Aldrich) drop wisely. The reaction mixture is stirred for 2 days at room temperature. 17 g (10 mmol) methyltriflate (Aldrich)are added and the reaction mixture is stirred at 40°C for 20 h. The reaction mixture is cooled to 20 °C and diethylether is added. The desired compound precipitates and the solvent is decanted and the solid is washed extensively (ten times) with large amounts of diethylether. The solid is dried by use of oil pump vacuum and purified by (C-18) RP-column chromatography (acetonitril/water - gradient 1:99 to 80:20). The desired compound **18b** is obtained in 86% yield (6,78 g, 18,1 mmol).
MS-ESI: 227/229 (M⁺, 78/21),

| Elementary analysis: | | | | |
|---|---|---|---|---|
| Calculated: | C 38.15% | H 4.00% | F 15.09% | N 3.71% |
| Determined: | C 38.17% | H 4.03% | F 15.05% | N 3.70% |

### c) Synthesis of (3-carboxy-4-chloro-phenyl)-trimethyl-ammonium trifluoromethanesulfonate (18c).

A solution of 0.5 g (1,32 mmol) **18b**, 12 ml dest. water and 12 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **18c** is obtained in 98% yield (471 mg, 1,3 mmol) and crude compound **18c** is used for the next step without purification.
MS-ESI: 214/216 (M⁺, 89/51),

| Elementary analysis: | | | | |
|---|---|---|---|---|
| Calculated: | C 36.32% | H 3.60% | F 15.67% | N 3.85% |
| Determined: | C 36.37% | H 3,63% | F 15.61% | N 3,83% |

### d) Synthesis of (5-Trimethytammonium-2-chloro-benzoyl)-Val-βAla-Arg-Gly-NH₂- triflate salt (18d).

To a stirred solution of 73.0 mg (0.2 mmol) **18c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Arg(Pbf)-Gly-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 73.0 mg (0.2 mmol) **18c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **(18d)** in 31 % yield - 15,3 mg (0.020 mmol).
MS-ESI: 611/613 (M⁺ +1, 100/41).

### e) Synthesis of [¹⁸]-(5-Fluoro-2-chloro-benzoyl)-Val-ßAla-Arg-Gly-NH₂ (18e).

To a Wheaton vial (5ml) charged with Kryptofix 222 (5mg), potassium carbonate (1mg in 500µl water) and MeCN (1.5ml) the fluorine containing water (384 MBq, 35µl) is added. The solvent is removed by heating at 120°C for 10 mins under a stream of nitrogen. Anhydrous MeCN (1ml) is added and evaporated as before. This step is repeated again. A solution of **18d** (2mg) in anhydrous DMSO (300µl) is added. After heating at 80°C for 20 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **18e** is confirmed by co-injection with the non-radioactive F-19 fluoro standard on the Econsphere analytical HPLC.

### Example 19

### a) 5-Dimethylamino-2-nitro-benzoic acid methyl ester (19a)

To a stirred solution of 4,48 g (22.5 mmol) 5-Fluoro-5-nitro-benzoic acid methyl ester (Rarechem) and 60.0 ml dimethylsulphoxid are added 2.23 g (27.0 mmol) dimethylamine hydrochloride and 6.54 g (47.3 mmol) potassium carbonate. The reaction mixture is stirred for 8h at 60°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **19a** is obtained in 69 % yield (3,49 g, 15,5 mmol).
MS-ESI: 225 (M⁺ +1, 52).

| | | | |
|---|---|---|---|
| Elementary analysis: | C 53.57% | H 5.39% | N 12.49% |
| Determined: | C 53.61% | H 5.40% | N 12,47% |

### b) Trifluoro-methanesulfonate(3-methoxycarbonyl-4-nitro-phenyl)-trimethyl -ammonium (19b)

To a stirred solution of 2,80 g (12,5 mmol) **19a** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **19b** is obtained in 71% yield (3,44 g, 8,88 mmol).
MS-ESI: 239 (M⁺, 69),

| Elementary analysis: | | | | |
|---|---|---|---|---|
| Calculated: | C 37.12% | H 3.89% | F 14.68% | N 7.21% |
| Determined: | C 37.14% | H 3.91% | F 14.66% | N 7.20% |

### c) Trifluoro-methanesulfonate(3-carboxy-4-nitro-phenyl)-trimethyl-ammonium (19c).

A solution of 2,46 g (6,92 mmol) **19b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 93% yield (2,41 g; 6.44 mmol) and crude compound **19c** is used for the next step without purification.
MS-ESI: 225 (M⁺ , 96),

| Elementary analysis: | | | | |
|---|---|---|---|---|
| Calculated: | C 35.30% | H 3.50% | F 15.23% | N 7.48% |
| Determined: | C 35.34% | H 3,52% | F 15.23% | N 7,47% |

### d) Synthesis of (2-Nitro-5-trimethylammonium-benzoyl)-Val-βAla-Phe-Gly-NH₂- triflate salt (19d).

To a stirred solution of 75,0 mg (0.2 mmol) **19c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Phe-Gly-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 75,0 mg (0.2 mmol) **19c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent **19d** is obtained in 42% yield (20,8 mg, 0.0273 mmol).
MS-ESI: 614 (M⁺ , 100).

### e) Synthesis of [¹⁸F]-(5-Fluoro-2-nitro-benzoyl)-Val-ßAla-Phe-Gly-NH₂ (19e).

To a Wheaton vial (5ml) charged with Kryptofix 222 (5mg), potassium carbonate (1 mg in 500µl water) and MeCN (1.5ml) the fluorine containing water (311 MBq, 35µl) is added. The solvent is removed by heating at 120°C for 10 mins under a stream of nitrogen. Anhydrous MeCN (1ml) is added and evaporated as before. This step is repeated again. A solution of **19d** (2mg) in anhydrous DMSO (300µl) is added. After heating at 70°C for 12 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **19e** is confirmed by co-injection with the non-radioactive F-19 fluoro standard on the Econsphere analytical HPLC.

### Example 20

### a) 2-Chloro-4-dimethylamino-5-methanesulfonyl-benzoic acid methyl ester (20a)

To a stirred solution of 6,00 g (22.5 mmol) 2-Chloro-4-fluoro-5-methanesulfonyl-benzoic acid methyl ester (J. Med. Chem.; 40; 13; 1997; 2017-2034) and 60.0 ml dimethylsulphoxid are added 2.23 g (27.0 mmol) dimethylamine hydrochloride and 6.54 g (47.3 mmol) potassium carbonate. The reaction mixture is stirred for 15h at 65°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **20a** is obtained in 59 % yield (3,87 g, 13,3 mmol).
MS-ESI: 292/294 (M⁺ +1, 69/23).

| | | | |
|---|---|---|---|
| Elementary analysis: | C 45.29% | H 4.84% | N 4.80% |
| Determined: | C 45.31% | H 4.86% | N 4,78% |

### b) Trifluoro-methanesulfonate(5-chloro-2-methanesulfonyl-4-methoxycarbonyl-phenyl)-trimethyl-ammonium (20b)

To a stirred solution of 3,65 g (12,5 mmol) **20a** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **20b** is obtained in 58% yield (3,31 g, 7,25 mmol).
MS-ESI: 307 (M⁺, 100),

| Elementary analysis: | | | | |
|---|---|---|---|---|
| Calculated: | C 34.25% | H 3.76% | F 12.50% | N 3.07% |
| Determined: | C 34.24% | H 3.80% | F 12.47% | N 3.06% |

### c) Trifluoro-methanesulfonate(4-carboxy-5-chloro-2-methanesulfonyl-phenyl)-trimethyl-ammonium (20c).

A solution of 3,16 g (6,92 mmol) **20b**, 60 ml dest. water and 60 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 83% yield (2,53 g; 5.74 mmol) and crude compound **20c** is used for the next step without purification.
MS-ESI: 293 (M⁺ , 48),

| Elementary analysis: | | | | |
|---|---|---|---|---|
| Calculated: | C 32.62% | H 3.42% | F 12.90% | N 3.17% |
| Determined: | C 32.64% | H 3,44% | F 12.89% | N 3,16% |

### d) Synthesis of (2-Chloro-5-methanesulfonyl-4-trimethylammonium-benzoyl)-Val-flAla-Phe-Gly-NH₂- triflate salt (20d).

To a stirred solution of 88,4 mg (0.2 mmol) **20c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Phe-Gly-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 88,4 mg (0.2 mmol) **20c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound H-Val-ßAla-Phe-Gly-NH₂ and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent. The desired compound **20d** is obtained in 43% yield (23,2 mg, 280 mmol).
MS-ESI: 681/683 (M⁺ , 100).

### e) Synthesis of [¹⁸F]-(2-Chloro-4-fluoro-5-methanesulfonyl-benzoyl)-Val-ßAla-Phe-Gly-NH₂ (20e).

To a Wheaton vial (5ml) charged with Kryptofix 222 (5mg), potassium carbonate (1mg in 500µl water) and MeCN (1.5ml) the fluorine containing water (322 MBq, 35µl) is added. The solvent is removed by heating at 120°C for 10 mins under a stream of nitrogen. Anhydrous MeCN (1ml) is added and evaporated as before. This step is repeated again. A solution of **20d** (2mg) in anhydrous DMSO (300µl) is added. After heating at 90°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **20e** is confirmed by co-injection with the non-radioactive F-19 fluoro standard on the Econsphere analytical HPLC.

### Example 21

### a) Synthesis of 2-Cyano-5-fluoro-benzoic acid methyl ester (21a).

To a stirred suspension of 16,0 g (96,9 mmol) 2-Cyano-5-fluoro-benzoic acid (Apollo) and 161 ml methanol are added 30.4 g (387,6 mmol) acetyl chloride drop wisely at 0°C.

The reaction mixture is stirred over night, filtered and concentrated. The residue is diluted with dichloromethane, washed with diluted sodium hydrogen carbonate solution, dried with sodium sulphate and concentrated. The residue is purified by column chromatography (hexane : ethylacetate). The desired product **21a** is obtained in 86.0 % yield (14,9 g; 83.3 mmol)
MS-ESI: 180 (M⁺ +1,100),

| Elementary analysis: | | | | |
|---|---|---|---|---|
| Calculated: | C 60.34% | H 3.38% | F 10.60% | N 7.82% |
| Determined: | C 60.41% | H 3,39% | F 10.58% | N 7,79% |

### b) Synthesis of 2-Cyano-5-dimethylamino-benzoic acid methyl ester (21b).

To a stirred solution of 4.03 g (22,5 mmol) **21a** and 60.0 ml dimethylsulphoxid are added 2.23 g (27.0 mmol) dimethylamine hydrochloride and 6.54 g (47.3 mmol) potassium carbonate. The reaction mixture is stirred for 15h at 65°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **21b** is obtained in 89.0 % yield (4.09 g, 20.0 mmol).
MS-ESI: 205 (M⁺ +1, 100),

| Elementary analysis: | | | |
|---|---|---|---|
| Calculated: | C 64.69% | H 5.92% | N 13.72% |
| Determined: | C 64.75% | H 5,94% | N 13.68% |

### c) Trifluoro-methanesulfonate(4-cyano-3-methoxycarbonyl-phenyl)-trimethyl -ammonium (21c).

To a stirred solution of 2.55 g (12.5 mmol) **21b** and 50 ml dichloromethane are added 20,5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **21c** is obtained in 88% yield (4,05 g, 11.0 mmol).
MS-ESI: 219 (M⁺ , 71),

| Elementary analysis: | | | | |
|---|---|---|---|---|
| Calculated: | C 42.39% | H 4.10% | F 15.47% | N 7.61% |
| Determined: | C 42.41% | H 4.13% | F 15.45% | N 7.60% |

### d) Synthesis of Trifluoro-methanesulfonate(3-carboxy-4-cyano-phenyl)-trimethylammonium (21d).

A solution of 4,01 g (10.9 mmol) **21c,** 95 ml dest. water and 95 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **21d** is obtained in 96% yield (3,70 g, 10,5 mmol) and crude compound **21d** is used for the next step without purification.
MS-ESI: 205 (M⁺, 76),

| Elementary analysis: | | | | |
|---|---|---|---|---|
| Calculated: | C 40.68% | H 3.70% | F 16.09% | N 7.91% |
| Determined: | C 40.70% | H 3,72% | F 16.07% | N 7,90% |

### e) Synthesis of peptide (5-Trimethylammonium-2-cyano-benzoyl)-Val-ßAla-Arg-Gly-NH₂ - triflate salt (21e).

To a stirred solution of 70.8 mg (0.2 mmol) **21d** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Arg(Pbf)-Gly-NH2 (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **21d** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent. The desired compound **21e** is obtained in 38% yield (18,6 mg, 0.0247 mmol).
MS-ESI: 603 (M⁺, 100),

### f) Synthesis of [¹⁸F]-(5-Fluoro-2-cyano-benzoyl)-Val-ßAla-Arg-Gly-NH₂ (21f).

To a Wheaton vial (5ml) charged with Kryptofix 222 (5mg), potassium carbonate (1mg in 500µl water) and MeCN (1.5ml) the fluorine containing water (345 MBq, 35µl) is added. The solvent is removed by heating at 120°C for 10 mins under a stream of nitrogen. Anhydrous MeCN (1ml) is added and evaporated as before. This step is repeated again. A solution of **21e** (2mg) in anhydrous DMSO (300µl) is added. After heating at 90°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **21f** is confirmed by co-injection with the non-radioactive F-19 fluoro standard on the Econsphere analytical HPLC.

### Example 22

### a) Synthesis of 2-Chloro-4,5-difluoro-benzoic acid methyl ester (22a).

To a stirred suspension of 5,0 g (26 mmol) 2-Chloro-4,5-difluoro-benzoic acid (Apollo) and 50 ml methanol are added 7,41 ml (104 mmol) acetyl chloride drop wisely at 0°C. The reaction mixture is stirred over night, filtered and concentrated. The residue is diluted with dichloromethane, washed with diluted sodium hydrogen carbonate solution, dried with sodium sulphate and concentrated. The residue is purified by column chromatography (hexane : ethylacetate). The desired product **22a** is obtained in 84% yield (4,51 g, 21.8 mmol).
MS-ESI: 207 / 209 (M⁺ +1, 64 / 22).

| Elementary analysis: | | | |
|---|---|---|---|
| Calculated: | C 46.51% | H 2.44% | F 18.39% |
| Determined: | C 46.59% | H 2.46% | F 18.35% |

### b) 2-Chloro-4-dimethylamino-5-fluoro-benzoic acid methyl ester (22b).

To a stirred solution of 23.1 g (112 mmol) **22a** and 231 ml dimethylsulphoxid are added 10.0 g (123 mmol) dimethylamine hydrochloride and 32.4 g (234 mmol) potassium carbonate. The reaction mixture is stirred for 24h at 60°C in an autoclave and is reduced with high vacuum rotation evaporator at 65°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude is purified by column chromatography and the desired product **22b** is obtained in 89,5% yield (23.1 g 100 mmol).
MS-ESI: 232 / 234 (M⁺ +1, 55 / 18).

| Elementary analysis: | | | | |
|---|---|---|---|---|
| Calculated: | C 51.85% | H 4.79% | F 8.20% | N 6.05% |
| Determined: | C 51.89% | H 4,81% | F 8,18% | N 6,03% |

### c) Trifluoro-methanesulfonate(5-chloro-2-fluoro-4-methoxycarbonyl-phenyl)-trimethylammonium (22c).

To a stirred solution of 7.06 g (30.5 mmol) **22b** and 100 ml dichloroethane are added 50 g (305 mmol) methyltriflate (Aldrich) drop wisely. The reaction mixture is stirred for 24 hours at 90°C and then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **22c** is obtained in 91.1% yield (11.0 g; 27.8 mmol).
MS-ESI: 246 / 248 (M⁺, 100 / 32).

| | | | | |
|---|---|---|---|---|
| Calculated: | C 36.42% | H 3.57% | F 19.20% | N 3.54% |
| Determined: | C 36,46% | H 3.58% | F 19.18% | N 3.51% |

### d) Synthesis of Trifluoro-methanesulfonate(4-carboxy-5-chloro-2-fluoro-phenyl)-trimethyl-ammonium (22d).

A solution of 2.0 g (5.05 mmol) **22c**, 45 ml dest. water and 45 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude product **22d** is obtained and crude compound **22d** is used for the next step without purification. The desired crude compound **22d** is obtained in 76% yield (1,46 g, 3,84 mmol).
MS-ESI: 232 / 234 (M⁺, 68/21).

| | | | | |
|---|---|---|---|---|
| Calculated: | C 34.61% | H 3.17% | F 19.91% | N 3.67% |
| Determined: | C 34,66% | H 3.19% | F 19.94% | N 3.66% |

### e) Synthesis of peptide (4-Trimethylammonium-2-chloro-5-fluoro-benzoyl)-Val-βAla-Phe-Gly-NH₂-triflate salt (22e).

To a stirred solution of 76 mg (0.2 mmol) **22d** in 1.5 ml dichloromethane and 0.25 ml dimethylformamid are added 0.031 ml (0.2 mmol) diisopropylcarbodiimid. The solution is added to 0.065 mmol Rink-resin-bound H-Val-ßAla-Phe-Gly-NH₂ (loading 0.68 mmol/g) which is prepared by standard protocol. The mixture is shaken intensively for 4h. The mixture is filtered and washed with dimethylformamide The coupling step is repeated. Thus, to a stirred solution of 76 mg (0.2 mmol) **22d** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound H-Val-βAla-Phe-Gly-NH₂ and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water : acetonitril gradient and 0.1% trifluoro acetic acid as co-solvent to obtain **22e** in 37% yield (18.6 mg, 0,0241 mmol).
MS-ESI: 620/622 (M⁺ ,100/34).

### f) Synthesis of [¹⁸F]-(4-Trimethylammonium-2-chloro-5-fluoro-benzoyl)-Val-ßAla-Phe-Gly-NH₂ (22f).

To a Wheaton vial (5ml) charged with Kryptofix 222 (5mg), potassium carbonate (1 mg in 500µl water) and MeCN (1.5ml) the fluorine containing water (319 MBq, 33µl) is added. The solvent is removed by heating at 120°C for 10 mins under a stream of nitrogen. Anhydrous MeCN (1ml) is added and evaporated as before. This step is repeated again. A solution of **22e** (2mg) in anhydrous DMSO (300µl) is added. After heating at 90°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **22f** is confirmed by co-injection with the non-radioactive F-19 fluoro standard on the Econsphere analytical HPLC.

### Example 23

### Synthesis of (4-Trimethylammonium-3-cyano-benzoyl)-Gly-Tyr-ßAla-Val-NH₂ , (23a).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Gly-Tyr(OtBu)-βAla-Val-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **23a** in 40% yield (19,8 mg (0.0176 mmol)).
MS-ESI: 609 (M⁺, 67).

### Synthesis of [¹⁸F]-4-Fluoro-3-cyano-benzoyl)-Gly-Tyr-ßAla-Val-NH₂, (23b).

To a Wheaton vial (5ml) charged with Kryptofix 222 (5mg), potassium carbonate (1mg in 500µl water) and MeCN (1.5ml) the fluorine containing water (356 MBq, 35µl) is added. The solvent is removed by heating at 120°C for 10 mins under a stream of nitrogen. Anhydrous MeCN (1ml) is added and evaporated as before. This step is repeated again. A solution of **23a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **23b** is confirmed by co-injection with the non-radioactive F-19 fluoro standard on the Econsphere analytical HPLC.

### Example 24

### Synthesis of (4-Trimethylammonium-3-cyano-benzoyl)-Ava-His(π-Me)-Sta-Leu-NH₂-triflate salt, (24a).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Ava-His(π-Me)-Sta-Leu-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **24a** in 38% yield (21.6 mg (0.023 mmol)).
MS-ESI: 727 (M⁺, 77).

### Synthesis of [¹⁸F]-4-Fluoro-3-cyano-benzoyl)-Ava-His(π-Me)-Sta-Leu-NH₂, (24b).

To a Wheaton vial (5ml) charged with Kryptofix 222 (5mg), potassium carbonate (1 mg in 500µl water) and MeCN (1.5ml) the fluorine containing water (377 MBq, 35µl) is added. The solvent is removed by heating at 120°C for 10 mins under a stream of nitrogen. Anhydrous MeCN (1ml) is added and evaporated as before. This step is repeated again. A solution of **24a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **24b** is confirmed by co-injection with the non-radioactive F-19 fluoro standard on the Econsphere analytical HPLC.

### Example 25

### Synthesis of (4-Trimethylammonium-3-cyano-benzoyl)-N-MeGly-His(π-Me)-Sta-Leu-NH₂-triflate salt, (25a).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-N-MeGly-His(Tr)-Sta-Leu-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **25a** in 29% yield (16,0 mg (0.0189 mmol)).
MS-ESI: 698 (M⁺, 75).

### Synthesis of [¹⁸F]-4-Fluoro-3-cyano-benzoyl)-N-MeGly-His(π-Me)-Sta-Leu-NH₂, (25b).

To a Wheaton vial (5ml) charged with Kryptofix 222 (5mg), potassium carbonate (1mg in 500µl water) and MeCN (1.5ml) the fluorine containing water (382 MBq, 35µl) is added. The solvent is removed by heating at 120°C for 10 mins under a stream of nitrogen. Anhydrous MeCN (1ml) is added and evaporated as before. This step is repeated again. A solution of **25a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **25b** is confirmed by co-injection with the non-radioactive F-19 fluoro standard on the Econsphere analytical HPLC.

### Example 26

### Synthesis of (4-Trimethylammonium-3-cyano-benzoyl)-Val-ßAla-Arg-Gly-NH₂-triflate salt, (26a).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Arg(Pbf)-Gly-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **26a** in 47% yield (20.3 mg, 0.0305 mmol).
(19,5 mg (0.026 mmol)).
MS-ESI: 603 (M⁺, 100).

### Synthesis of [¹⁸F]-4-Fluoro-3-cyano-benzoyl)-Val-ßAla-Arg-Gly-NH₂, (26b).

To a Wheaton vial (5ml) charged with Kryptofix 222 (5mg), potassium carbonate (1mg in 500µl water) and MeCN (1.5ml) the fluorine containing water (344 MBq, 35µl) is added. The solvent is removed by heating at 120°C for 10 mins under a stream of nitrogen. Anhydrous MeCN (1ml) is added and evaporated as before. This step is repeated again. A solution of **26a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **26b** is confirmed by co-injection with the non-radioactive F-19 fluoro standard on the Econsphere analytical HPLC.

### Example 27

### Synthesis of (4-Trimethylammonium-3-cyano-benzoyl)-Val-ßAla-His(Π-Me)-Gly-NH₂ triflate salt, (27a).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-His(II-Me)-Gly-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **27a** in 33% yield (16,0 mg (0.0215 mmol)).
MS-ESI: 597 (M⁺, 100).

### Synthesis of [¹⁸F]-4-Fluoro-3-cyano-benzoyl)-Val-ßAla-His(π-Me)-Gly-NH₂, (27b).

To a Wheaton vial (5ml) charged with Kryptofix 222 (5mg), potassium carbonate (1mg in 500µl water) and MeCN (1.5ml) the fluorine containing water (367 MBq, 35µl) is added. The solvent is removed by heating at 120°C for 10 mins under a stream of nitrogen. Anhydrous MeCN (1ml) is added and evaporated as before. This step is repeated again. A solution of **27a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **27b** is confirmed by co-injection with the non-radioactive F-19 fluoro standard on the Econsphere analytical HPLC.

### Example 28

### Synthesis of (4-Trimethylammonium-3-cyano-benzoyl)-Val-βAla-His(π-Me)-Leu-NH₂ triflate salt, (28a).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-His(π-Me)-Leu-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **28a** in 57% yield (29,8 mg (0.037 mmol)).
MS-ESI: 654 (M⁺, 100).

### Synthesis of [¹⁸F]-4-Fluoro-3-cyano-benzoyl)-Val-ßAla-His(π-Me)-Leu-NH₂, (28b).

To a Wheaton vial (5ml) charged with Kryptofix 222 (5mg), potassium carbonate (1mg in 500µl water) and MeCN (1.5ml) the fluorine containing water (356 MBq, 35µl) is added. The solvent is removed by heating at 120°C for 10 mins under a stream of nitrogen. Anhydrous MeCN (1ml) is added and evaporated as before. This step is repeated again. A solution of **28a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **28b** is confirmed by co-injection with the non-radioactive F-19 fluoro standard on the Econsphere analytical HPLC.

### Example 29

### Synthesis of (4-Trimethylammonium-3-cyano-benzoyl)-Val-ßAla-Phe-Gly-NH₂ triflate salt, (29a).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Phe-Gly-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **29a** in 46% yield (22,2 mg (0.0299 mmol)).
MS-ESI: 593 (M⁺, 100).

### Synthesis of [¹⁸F]-(4-Fluoro-3-cyano-benzoyl)-Val-ßAla-Phe-Gly-NH₂, (29b).

To a Wheaton vial (5ml) charged with Kryptofix 222 (5mg), potassium carbonate (1mg in 500µl water) and MeCN (1.5ml) the fluorine containing water (333 MBq, 35µl) is added. The solvent is removed by heating at 120°C for 10 mins under a stream of nitrogen. Anhydrous MeCN (1ml) is added and evaporated as before. This step is repeated again. A solution of **29a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **29b** is confirmed by co-injection with the non-radioactive F-19 fluoro standard on the Econsphere analytical HPLC.

### Example 30

### Synthesis of (4-Trimethylammonium-3-cyano-benzoyl)-Val-ßAla-Trp-Gly-NH₂-triflate salt, (30a).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Trp(Boc)-Gly-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **30a** in 41% yield (20,8 mg (0.021 mmol)).
MS-ESI: 632 (M⁺, 100).

### Synthesis of [¹⁸F]-4-Fluoro-3-cyano-benzoyl)-Val-ßAla-Trp-Gly-NH₂, (30b).

To a Wheaton vial (5ml) charged with Kryptofix 222 (5mg), potassium carbonate (1mg in 500µl water) and MeCN (1.5ml) the fluorine containing water (368 MBq, 35µl) is added. The solvent is removed by heating at 120°C for 10 mins under a stream of nitrogen. Anhydrous MeCN (1ml) is added and evaporated as before. This step is repeated again. A solution of **30a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18, 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **30b** is confirmed by co-injection with the non-radioactive F-19 fluoro standard on the Econsphere analytical HPLC.

### Example 31

### Synthesis of (4-Trimethylammonium-3-cyano-benzoyl)-Val-ßAla-Tyr-Gly-NH₂ triflate salt, (31a).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Tyr(OtBu)-Gly-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **31a** in 36% yield (17,7 mg (0.0234 mmol)).
MS-ESI: 609 (M⁺, 100).

### Synthesis of [¹⁸F]-4-Fluoro-3-cyano-benzoyl)-Val-ßAla-Tyr-Gly-NH₂, (31b).

To a Wheaton vial (5ml) charged with Kryptofix 222 (5mg), potassium carbonate (1mg in 500µl water) and MeCN (1.5ml) the fluorine containing water (339 MBq, 35µl) is added. The solvent is removed by heating at 120°C for 10 mins under a stream of nitrogen. Anhydrous MeCN (1ml) is added and evaporated as before. This step is repeated again. A solution of **31a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **31b** is confirmed by co-injection with the non-radioactive F-19 fluoro standard on the Econsphere analytical HPLC.

### Example 32

### a) Synthesis of (4-Trimethylammonium-3-trifluormethyl-benzoyl)-Val-ßAla-His(π-Me)-Gly-NH₂- triflate salt (32a).

To a stirred solution of 79,4 mg (0.2 mmol) **12c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-His(π-Me)-Gly-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 79.4 mg (0.2 mmol) **12c** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product (**32a**) in 59% yield (24,2 mg (0.0384 mmol)).
MS-ESI: 598 (M⁺, 88)

### b) Synthesis of [¹⁸F]-4-Fluoro-3-cyano-benzoyl)-Val-ßAla-His(π-Me)-Gly-NH₂, (32b).

To a Wheaton vial (5ml) charged with Kryptofix 222 (5mg), potassium carbonate (1mg in 500µl water) and MeCN (1.5ml) the fluorine containing water (318 MBq, 35µl) is added. The solvent is removed by heating at 120°C for 10 mins under a stream of nitrogen. Anhydrous MeCN (1ml) is added and evaporated as before. This step is repeated again. A solution of **32a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **32b** is confirmed by co-injection with the non-radioactive F-19 fluoro standard on the Econsphere analytical HPLC.

### Example 33

### a) Synthesis of (4-Trimethylammonium-3-cyano-benzoyl)-Val-ßAla-Lys-Gly-NH₂- triflate salt (33a).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Lys(Boc)-Gly-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **33a** in 52% yield (24,5 mg (0.0338 mmol)).
MS-ESI: 575 (M⁺, 100).

### b) Synthesis of [¹⁸F]-4-Fluoro-3-cyano-benzoyl)-Val-βAla-Lys-Gly-NH₂, (33b).

To a Wheaton vial (5ml) charged with Kryptofix 222 (5mg), potassium carbonate (1mg in 500µl water) and MeCN (1.5ml) the fluorine containing water (301 MBq, 35µl) is added. The solvent is removed by heating at 120°C for 10 mins under a stream of nitrogen. Anhydrous MeCN (1ml) is added and evaporated as before. This step is repeated again. A solution of **33a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **33b** is confirmed by co-injection with the non-radioactive F-19 fluoro standard on the Econsphere analytical HPLC.

### Example 34

### a) Synthesis of (4-Trimethylammonium-3-cyano-benzoyl)-Val-ßAla-Met-Gly-NH₂- triflate salt (34a).

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-Met-Gly-NH₂ (loading 0.68 mmol/g) which is prepared by standard protocol. The mixture is shaken intensively for 4h. The mixture is filtered and washed with dimethylformamide.. The coupling step is repeated. Thus, to a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed peptide resin and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **34a** in 37% yield (17,4 mg (0.024 mmol)).
MS-ESI: 577 (M⁺, 100).

### b) Synthesis of [¹⁸F]-4-Fluoro-3-cyano-benzoyl)-Val-ßAla-Met-Gly-NH₂, (34b).

To a Wheaton vial (5ml) charged with Kryptofix 222 (5mg), potassium carbonate (1 mg in 500µl water) and MeCN (1.5ml) the fluorine containing water (383 MBq, 35µl) is added. The solvent is removed by heating at 120°C for 10 mins under a stream of nitrogen. Anhydrous MeCN (1ml) is added and evaporated as before. This step is repeated again. A solution of **34a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **34b** is confirmed by co-injection with the non-radioactive F-19 fluoro standard on the Econsphere analytical HPLC.

### Example 35

### a) Synthesis of (3-Cyano-4-fluoro-benzenesulfonyl)-methyl-amino]-acetic acid methyl ester (35a)

To a suspension of 6.90 g (0.05 mol) sarcosin hydrochloride and 19.5 g (0.15 mol) diisopropylethyl amin in 70.0 ml dichloromethane are added 11.5 g (0.055 mol) 3-cyano-4-fluoro-benzenesulfonyl chloride (Aldrich) in 50.0 ml dichloromethane dropwisely at 0°C. The suspension is stirred for 4h. The suspension is poured on 150 ml stirred ice/water mixture. The water phase is separated and extracted twice with dichlormethane. The combined dichloromethane phases are washed twice with diluted hydrogen chloride solution and subsequently with sodium hydrogen carbonate solution, dried with sodium sulphate and concentrated. The oily crude product **35a** is purified by column chromatography (hexane : ethylacetate). The desired product **35a** is obtained in 58,2% yield (8.32 g ; 29.1 mmol)
MS-ESI: 287 (M⁺ +1, 100).

| | | | | |
|---|---|---|---|---|
| Elementary analysis: | C 46.15% | H 3.87% | F 6.64% | N 9.79% |
| Determined: | C 46.16% | H 3.88% | F 6.65% | N 9.80% |

### b) Synthesis of (3-Cyano-4-dimethylamino-benzenesulfonyl)-methyl-amino]-acetic acid methyl ester (35b)

To a stirred solution of 5.72 g (20.0 mmol) **35a** and 60 ml dimethylsulphoxid are added 2.03 g (24,7 mmol) dimethylamine hydrochloride and 5,97 g (43.2 mmol) potassium carbonate. The reaction mixture is stirred over night and is reduced with high vacuum rotation evaporator at 60°C. The residue is diluted with dichloromethane, washed twice with water. The combined water phases are extracted with dichloromethane. The combined dichloromethane phases are washed with diluted sodium hydrogen carbonate solution, dried with sodium sulphate and concentrated. The oily crude product 35b is obtained in 95% yield (5.92 g, 19.9 mmol) and is used for the next step without purification.
MS-ESI: 312 (M⁺ +1, 100).

| | | | |
|---|---|---|---|
| Elementary analysis: | C 50.15% | H 5.50% | N 13.50% |
| Determined: | C 50.18% | H 5.52% | N 13.48% |

### c) Synthesis of Trifluoro-methanesulfonate[2-cyano-4-(methoxycarbonylmethyl-methylsulfamoyl)-phenyl]-trimethyl-ammonium (35c)

To a stirred solution of 3.89 g (12.5 mmol) **35b** and 50 ml dichloromethane are added 20.5 g (125 mmol) methyltriflate (Aldrich) drop wisely. The reaction mixture is refluxed for 2 days then cooled to room temperature. Diethylether is added. The desired compound precipitates and the solvent is decanted. The solid is washed extensively (ten times) with large amounts of diethylether. The solid is dried by use of oil pump vacuum and purified by (C-18) RP-column chromatography (acetonitril/water - gradient 1:99 to 80:20). The desired compound **35c** is obtained in 43 % yield (2,55 g, 5,375 mmol).
MS-ESI: 326 (M⁺, 100).

| | | | | |
|---|---|---|---|---|
| Elementary analysis: | C 37.89% | H 4.24% | F 11.99% | N 8.84% |
| Determined: | C 37.92% | H 4.26% | F 11.96% | N 8.86% |

### d) Trifluoro-methanesulfonate[4-(carboxymethyl-methyl-sulfamoyl)-2-cyano-phenyl]-trimethyl-ammonium (35d)

A solution of 2,38g (5,0 mmol) **35c**, 50 ml dest. water and 50 ml trifluoroacetic acid is refluxed for 2 days. The reaction mixture is evaporated, dried by use of oil pump vacuum over night and treated with diethyl ether. The resulting solid is filtered, washed extensively with diethyl ether and dried by oil pump vacuum. The solid crude is obtained in 79% yield (1.82 g; 3,95 mmol) and crude compound **35d** is used for the next step without purification.
MS-ESI: 312 (M⁺ , 100).

| | | | | |
|---|---|---|---|---|
| Elementary analysis: | C 36.44% | H 3.93% | F 12.35% | N 9.11% |
| Determined: | C 36.47% | H 3.95% | F 12.33% | N 9.10% |

### e) Synthesis of (4-Trimethylammonium-3-cyano-benzenesulfonyl)-Gly-Val-ßAla-His(π-Me)-Gly-NH₂- triflate salt (35e)

To a stirred solution of 82,4 mg (0.2 mmol) **35d** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to 0.065 mmol Rink-resin-bound H-Val-ßAla-His(π-Me)-Gly-NH₂ (loading 0.68 mmol/g) which is prepared by standard protocol. The mixture is shaken intensively for 4h. The mixture is filtered and washed with dimethylformamide. The coupling step is repeated. Thus, to a stirred solution of 82 mg (0.2 mmol) **35d** in 1.5 ml dichloromethane and 0.25 ml dimethylformamid is added 65 mg (0.5 mmol) diisopropylethylamin and 0.031 ml (0.2 mmol) diisopropylcarbodiimid. The solution is added to the washed Rink-resin-bound peptide and the mixture is again shaken intensively for 4h. The mixture is filtered and washed extensively with dimethylformamide and dichloromethane. The resin is treated with a mixture of 0.85 ml trifluoroacetic acid, 0.05 ml distilled water, 0.05 ml phenol, 0.05 ml triisopropylsilane for 3h. The mixture is added in ca. 9 ml ice cold methyl tert-butyl ether. The solid is separated by centrifugation. Water is added to the solid and the supernatant is liophylized. The residue is purified by preparative RP-18 HPLC-MS with a water: acetonitril gradient and 0.1% trifluoro acetic acid as co-solvent to obtain the desired product **(35e)** in 55% yield - 30.5 mg (0.036 mmol).
MS-ESI: 705 (M⁺ , 100).

### f) Synthesis of [¹⁸F]-(4-Fluoro-3-cyano-benzenesulfonyl)-Val-ßAla-His(π-Me)-Gly-NH₂-triflate salt (35f)

To a Wheaton vial (5ml) charged with Kryptofix 222 (5mg), potassium carbonate (1 mg in 500µl water) and MeCN (1.5m1) the fluorine containing water (344 MBq, 33µl) is added. The solvent is removed by heating at 120°C for 10 mins under a stream of nitrogen. Anhydrous MeCN (1ml) is added and evaporated as before. This step is repeated again. A solution of **35e** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **35f** is confirmed by co-injection with the non-radioactive F-19 fluoro standard on the Econsphere analytical HPLC.

### Example 36

### a) Synthesis of N-(4-Trimethylammonium-3-cyano-benzoyl)-6-fluoro-dopamin - triflate salt (36a)

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is stirred for 20 min and 34 mg (0.2 mmol) symphatomimetic 6-fluorodopamin (J. Fluorine Chem.; 74; 1; 1995; 113-122, CAS Nr. 71144-39-3) is added. The reaction mixture is stirred intensively for 8 h. The reaction mixture is evaporated in vacuum, diluted with dichloromethane : iso-propanol mixture (10:1) and washed twice with water. The combined water phases are extracted with dichloromethane. The combined organic phases are washed with brine, dried with sodium sulphate and concentrated. The oily crude is purified by RP column chromatography (water : acetonitril gradient) and the desired product **36a** is obtained in 44 % yield (45 mg, 0.088 mmol).
MS-ESI: 358 (M⁺ , 100).

| | | | | |
|---|---|---|---|---|
| Elementary analysis: | C 47.34% | H 4.17% | F 14.98% | N 8.28% |
| Determined: | C 47.36% | H 4.19% | F 14.97% | N 8.27% |

### b) Synthesis of [¹⁸F]-N-(4-Fluoro-3-cyano-benzoyl)-6-fluoro-dopamin - triflate salt (36b)

To a Wheaton vial (5ml) charged with Kryptofix 222 (5mg), potassium carbonate (1mg in 500µl water) and MeCN (1.5ml) the fluorine containing water (356 MBq, 35µl) is added. The solvent is removed by heating at 120°C for 10 mins under a stream of nitrogen. Anhydrous MeCN (1ml) is added and evaporated as before. This step is repeated again. A solution of **36a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **36b** is confirmed by co-injection with the non-radioactive F-19 fluoro standard on the Econsphere analytical HPLC.

### Example 37

### a) Synthesis of N-(4-Trimethylammonium-3-cyano-benzoyl)-didemethyltamoxifentriflate salt (37a)

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is stirred for 20 min and 0.2 mmol estrogen antagonist didemethyltamoxifen (J. Pharm. Sci.; 82; 9; (1993); 927-933, CAS Nr. 80234-20-4) is added. The reaction mixture is stirred intensively for 8 h. The reaction mixture is evaporated in vacuum, diluted with dichloromethane and washed twice with water. The combined water phases are extracted with dichloromethane. The combined dichloromethane phases are washed with brine, dried with sodium sulphate and concentrated. The oily crude is purified by column chromatography and the desired product **37b** is obtained in 56 % yield (76 mg, 0.112 mmol)
MS-ESI: 531 (M⁺ , 100).

| | | | | |
|---|---|---|---|---|
| Elementary analysis: | C 63.61% | H 5.34% | F 8.38% | N 6.18% |
| Determined: | C 63.64% | H 5.35% | F 8.37% | N 6.17% |

### b) Synthesis of [¹⁸F]-N-(4-Fluoro-3-cyano-benzoyl) didemethyltamoxifen (37b)

To a Wheaton vial (5ml) charged with Kryptofix 222 (5mg), potassium carbonate (1mg in 500µl water) and MeCN (1.5ml) the fluorine containing water (337 MBq, 35µl) is added. The solvent is removed by heating at 120°C for 10 mins under a stream of nitrogen. Anhydrous MeCN (1ml) is added and evaporated as before. This step is repeated again. A solution of **37a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **37b** is confirmed by co-injection with the non-radioactive F-19 fluoro standard on the Econsphere analytical HPLC.

### Example 38

### a) Synthesis of N-(4-Trimethylammonium-3-cyano-benzoyl)-alaphen - triflate salt (38a)

To a stirred solution of 70.8 mg (0.2 mmol) **2e** in 1.5 ml dichloromethane and 0.25 ml dimethylformamid is added 65 mg (0.5 mmol) diisopropylethylamin and 0.031 ml (0.2 mmol) diisopropylcarbodiimid. The solution is stirred for 20 min and 49 mg (0.2 mmol) alaphen (Pharm.Chem.J.(Engl.Transl.); 9; 3; (1975); p. 158; CAS Nr. 15269-42-8) is added. The reaction mixture is stirred intensively for 8 h. The reaction mixture is evaporated in vacuum, diluted with dichloromethane:isopropanol mixture (10:1) and washed twice with water. The combined water phases are extracted with dichloromethane. The combined dichloromethane phases are washed with brine, dried with sodium sulphate and concentrated. The oily crude is purified by RP column chromatography and the desired product **38a** is obtained in 64 % yield (77 mg, 0.13 mmol).
MS-ESI: 394 (M⁺, 100)

| | | | | |
|---|---|---|---|---|
| Elementary analysis: | C 53.38% | H 5.84% | F 11.01% | N 8.12% |
| Determined: | C 53.41% | H 5.85% | F 11.00% | N 8.11% |

### b) Synthesis of [¹⁸F]-N-(4-Fluoro-3-cyano-benzoyl) alaphen (38b)

To a Wheaton vial (5ml) charged with Kryptofix 222 (5mg), potassium carbonate (1mg in 500µl water) and MeCN (1.5ml) the fluorine containing water (364 MBq, 35µl) is added. The solvent is removed by heating at 120°C for 10 mins under a stream of nitrogen. Anhydrous MeCN (1ml) is added and evaporated as before. This step is repeated again. A solution of **38a** (2mg) in anhydrous DMSO (300µl) is added. After heating at 50°C for 15 min. The crude reaction mixture is analyzed using an analytical HPLC (Column Zorbax SB C18, 50×4.6mm, 1.8µ, 2ml/min, solvent A: H2O, solvent B: MeCN, gradient: 5%-95% B in 7 mins or Column Econosphere C18 , 53×7mm, 3µ, 3ml/min (Alltech), solvent A: H2O + 0.1%TFA, solvent B: MeCN/Water 9/1 + 0.1% TFA, gradient: 5-95% B in 7 mins. The F-18 labeled product **38b** is confirmed by co-injection with the non-radioactive F-19 fluoro standard on the Econsphere analytical HPLC.

## Claims

1. A compound of Formula I 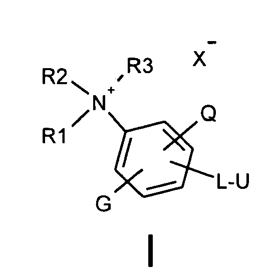 wherein
**L** is a bond, -CO-, -SO₂-, -(CH₂)_{d}-CO-, -SO-, or -C≡C-CO-, wherein the respective substituent can be in ortho, meta or para-position and d is an integer between 1 and 6,
**G** is selected from -F, -Cl, -Br, -I, -NO, -NO₂, -NR⁴COCF₃, -NR⁴SO₂CF₃, -N(CF₃)₂, -NHCSNHR⁴, -N(SO₂R⁵)₂, -N(O)=NCONH₂, -NR⁴CN, -NHCSR⁵, -N=C, -N=C(CF₃)₂, -N=NCF₃, -N=NCN, -NR⁴COR⁴, -NR⁴COOR⁵, -OSO₂CF₃, -OSO₂C₆H₅, -OCOR⁵, -ONO₂, -OSO₂R⁵, -O-C=CH₂, -OCF₂CF₃, -OCOCF₃, -OCN, -OCF₃, -C=N, -C(NO₂)₃, -COOR⁴, -CONR⁴R⁵, -CSNH₂, -CH=NOR⁴, -CH₂SO₂R⁴, -COCF₃, -CF₃, -CF₂Cl-CBr₃, -CCIF₂, -CCl₃, -CF₂CF₃, -C≡CR⁴, -CH=NSO₂CF₃, -CH₂CF₃, -COR⁵, -CH=NOR⁵, -CH₂CONH₂, -CSNHR⁵, -CH=NNHCSNH₂, -CH=NNHCONHNH₂, -C≡CF₃, -CF=CFCF₃, -CF₂-CF₂-CF₃, -CR⁴(CN)₂, -COCF₂CF₂CF₃, -C(CF₃)₃, -C(CN)₃, -CR⁴=C(CN)₂, -1-pyrryl, -C(CN)=C(CN)₂, -C-pyridyl, -COC₆H₅, -COOC₆H₅, -SOCF₃, -SO₂CF₃, -SCF₃, -SO₂CN, -SCOCF₃, -SOR⁵, -S(OR⁵), -SC≡CR⁴, -SO₂R⁵, -SSO₂R⁵, -SR⁵, -SSR⁴, -SO₂CF₂CF₃, -SCF₂CF₃, -S(CF₃)=NSO₂CF₃, -SO₂C₆H₅, -SO₂N(R⁵)₂, -SO₂C(CF₃)₃, -SC(CF₃)₃, -SO(CF₃)=NSO₂CF₃, -S(O)=NCF₃, -S(O)=NR⁵, -S-C=CH₂, -SCOR⁵, -SOC₆H₅, -P(O)C₃F₇, -PO(R⁵)₂, -PO(N(R⁵)₂)₂, -P(N(R⁵)₂)₂, -P(O)R⁵₂, and -PO(OR⁵)₂,
or another electron-drawing group, wherein the respective substituent can be in ortho, meta or para position,
**R¹, R²** and **R³** are independently from each other alkyl or aralkyl,
**R⁴** is independently from each other hydrogen or lower un-branched or branched alkyl,
**R⁵** is lower un-branched or branched alkyl,
**Q** is hydrogen, lower un-branched or branched alkyl, aryl, heteroaryl, -O-(C₁-C₄Alkyl), -CN, -halo, -SO₂-R⁴ or nitro, wherein respective substituent can be in *ortho, meta* or para position or a condensed aryl or heteroaryl,
**U** is a targeting agent, and
**X⁻** is CF₃S(O)₂O⁻, C₄F₉S(O)₂O⁻, iodide anion, bromide anion, chloride anion, perchlorate anion (ClO₄⁻), phosphate anion or other salts of inorganic or organic acids.

2. A compound according to claim 1 , wherein **R¹, R²** and **R³** are independently from each other aralkyl or lower alkyl, whereas at least two moieties of R¹, R², R³ are alkyl.

3. A compound according to claims 1 or 2, wherein **R¹** is selected from aralkyl and **R²** and **R³** are methyl.

4. A compound according to claims 1 to 3, wherein **R¹, R²** and **R³** are methyl.

5. A compound according to claims 1 to 4, wherein **X⁻** is CF₃S(O)₂O⁻ or C₄F₉S(O)₂O⁻.

6. A compound according to claims 1 to 5, wherein **X**⁻ is CF₃S(O)₂O⁻.

7. A compound of Formula II 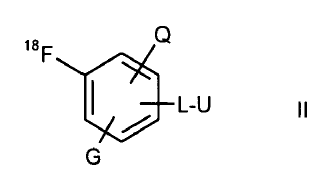 wherein:
L is a bond, -CO-, -SO₂-, -(CH₂)_{d}-CO-, -SO-, or -C≡C-CO-, wherein the respective substituent can be in ortho, meta or para-position and d is an integer between 1 and 6,
G is selected from -F, -Cl, -Br, -I, -NO, -NO₂, -NR⁴COCF₃, -NR⁴SO₂CF₃, -N(CF₃)₂, -NHCSNHR⁴, -N(SO₂R⁵) ₂ , -N(O)=NCONH₂, -NR⁴CN, -NHCSR⁵, -N=C, -N=C(CF₃)₂, -N=NCF₃, -N=NCN, -NR⁴COR⁴, -NR⁴COOR⁵, -OSO₂CF₃, -OSO₂C₆H₅, -OCOR⁵, -ONO₂, -OSO₂R⁵, -O-C=CH₂, -OCF₂CF₃, -OCOCF₃, -OCN, -OCF₃, -C=N, -C(NO₂)₃, -COOR⁴, -CONR⁴R⁵, -CSNH₂, -CH=NOR⁴, -CH₂SO₂R⁴, -COCF₃, -CF₃, -CF₂Cl-CBr₃, -CCIF₂, -CCl₃, -CF₂CF₃, -C≡CR⁴, -CH=NSO₂CF₃, -CH₂CF₃, -COR⁵, -CH=NOR⁵, -CH₂CONH₂, -CSNHR⁵, -CH=NNHCSNH₂, -CH=NNHCONHNH₂, -C≡CF₃, -CF=CFCF₃, -CF₂-CF₂-CF₃, -CR⁴(CN)₂, -COCF₂CF₂CF₃, -C(CF₃)₃, -C(CN)₃, -CR⁴=C(CN)₂, -1-pyrryl, -C(CN)=C(CN)₂, -C-pyridyl, -COC₆H₅, -COOC₆H₅, -SOCF₃, -SO₂CF₃, -SCF₃, -SO₂CN, -SCOCF₃, -SOR⁵, -S(OR⁵), -SC≡CR⁴, -SO₂R⁵, -SSO₂R⁵, -SR⁵, -SSR⁴, -SO₂CF₂CF₃, -SCF₂CF₃, -S(CF₃)=NSO₂CF₃, -SO₂C₆H₅, -SO₂N(R⁵)₂, -SO₂C(CF₃)₃, -SC(CF₃)₃, -SO(CF₃)=NSO₂CF₃, -S(O)=NCF₃, -S(O)=NR⁵, -S-C=CH₂, -SCOR⁵, -SOC₆H₅, -P(O)C₃F₇, -PO(R⁵)₂, -PO(N(R⁵)₂)₂, -P(N(R⁵)₂)₂, -P(O)R⁵₂, and -PO(OR⁵)₂,
or another electron-drawing group, wherein the respective substituent can be in ortho, meta or para position,
**R**⁴ is independently from each other hydrogen or lower un-branched or branched alkyl,
**R⁵** is lower un-branched or branched alkyl,
**Q** is hydrogen, lower un-branched or branched alkyl, aryl, heteroaryl, -O-(C₁-C₄Alkyl), -CN, -halo, -SO₂-R⁴ or nitro, wherein respective substituent can be in *ortho, meta* or *para* position or a condensed aryl or heteroaryl,
**U** is a targeting agent, and
**X⁻** is CF₃S(O)₂O⁻, C₄F₉S(O)₂O⁻, iodide anion, bromide anion, chloride anion, perchlorate anion (ClO₄⁻), phosphate anion or other salts of inorganic or organic acids.

8. A compound according to claims 1 to 7, wherein **U** is selected from peptide, small molecule or oligonucleotide.

9. A compound according to claims 1 to 8, wherein **U** is a peptide comprising from 4 to 100 amino acids.

10. A compound according to claims 1 to 9, wherein **U** is -NR⁴-peptide, or -(CH₂)ₙ-peptide, and n is an integer between 1 and 6.

11. A compound according to claims 1 to 10, wherein **L** is -CO-, -SO₂-, or -C=C-CO-, wherein the respective substituent can be in meta or para position.

12. A compound according to claims 1 to 11, wherein **L** is -CO-, or -SO₂, wherein the respective substituent can be in meta or para position.

13. A compound according to claims 1 to 12, wherein **G** is selected from -F, -Cl, -Br, -NO₂, -NR⁴SO₂R⁵, -NHCSNHR⁴, -NR⁴CN, -NR⁴SO₂CF₃, -N=C, -NR⁴COR⁴, -NR⁴COOR⁵, -OSO₂R⁵, -OCF₃, -C=N, -COOR⁴, -CONR⁴R⁵, -COCF₃, -CF₂CF₃, -C≡CR⁴, -COR⁵, -CH₂CONH₂, -CF₃, -C≡CF₃, -CF₂-CF₂-CF₃, -C(CN)=C(CN)₂, -COC₆H₅, -SO₂CF₃, -SCOCF₃, -SO₂R⁵, -SO₂CF₂CF₃, -SO₂C₆H₅, -SO₂N(R⁵) ₂, and -PO(OR⁵)₂, wherein the respective substituent can be in ortho, meta or para position.

14. A compound according to claims 1 to 13, wherein **G** is selected from -F, -Cl, -Br, -NO₂, -NR⁴SO₂R⁵, -NR⁴COR⁴, -NR⁴COOR⁵, -C≡N, -CONR⁴R⁵, -C≡CR⁴, -COR⁵, -CF₃, -COC₆H₅, -SO₂CF₃, -SO₂R⁵, -SO₂C₆H₅, -SO₂N(R⁵)₂, wherein the respective substituent can be in ortho, meta or para position.

15. A compound according to claims 1 to 14, wherein **R⁴** is independently from each other hydrogen or un-branched or branched C₁-C₄ alkyl.

16. A compound according to claims 1 to 15, wherein **R⁴** is independently from each other hydrogen or methyl.

17. A compound according to claims 1 to 16, wherein **R⁵** is un-branched or branched C₁-C₄ alkyl.

18. A compound according to claims 1 to 17, wherein **R⁵** is methyl.

19. A compound according to claims 1 to 18, wherein **Q** is hydrogen, C₁-C₄ alkyl, -O-(C₁-C₄Alkyl), -CN, -fluoro, -chloro, -bromo or nitro, wherein respective substituent can be in ortho, meta or para position.

20. A compound according to claims 1 to 19, wherein **Q** is hydrogen, methyl, -O-(methyl), -CN, -fluoro, -chloro, or nitro, wherein respective substituent can be in ortho, meta or para position.

21. A method of preparing a compound of Formula II **characterized by** reacting a compound of Formula I with an appropriate radiofluorination agent at appropriate reaction conditions.

22. A method according to claim 21, wherein the step of radiofluorination of a compound of Formula I is carried out at a temperature at or below 90°C.

23. A composition comprising a compound according to Formula I and a pharmaceutically acceptable carrier or diluent.

24. A composition comprising a compound according to Formula II and a pharmaceutically acceptable carrier or diluent.

25. A kit comprising a sealed vial containing a predetermined quantity of a compound according to Formula I.

26. A compound according to Formula II for use as medicament.

27. A compound according to Formula II for use as diagnostic imaging agent.

28. A compound according to Formula II for use as imaging agent for PET.

29. Use of a compound according to Formula I for the manufacturing of a medicament.

30. Use of a compound according to Formula II for the manufacturing of a medicament.
